(19) Europäisches Patentamt
European Patent Office
Office européen des brevets

(11) EP 2 881 381 B1

# (12) EUROPÄISCHE PATENTSCHRIFT

(45) Veröffentlichungstag und Bekanntmachung des Hinweises auf die Patenterteilung:
**27.06.2018 Patentblatt 2018/26**

(21) Anmeldenummer: **14191990.2**

(22) Anmeldetag: **06.11.2014**

(51) Int Cl.:
***A61K 8/37*** *(2006.01)* ***A61K 8/39*** *(2006.01)*
***A61K 8/86*** *(2006.01)* ***A61Q 1/14*** *(2006.01)*
***A61Q 15/00*** *(2006.01)* ***A61Q 5/02*** *(2006.01)*
***A61Q 19/10*** *(2006.01)* ***C07C 67/08*** *(2006.01)*
***C07C 69/52*** *(2006.01)* ***C07C 69/732*** *(2006.01)*

## (54) Polyglycerinpartialester, ihre Herstellung und Verwendung

Polyglycerine partial esters, their preparation and use

Esters partiels de polyglycérol, leur fabrication et leur utilisation

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.12.2013 DE 102013224957**

(43) Veröffentlichungstag der Anmeldung:
**10.06.2015 Patentblatt 2015/24**

(73) Patentinhaber: **Evonik Degussa GmbH**
**45128 Essen (DE)**

(72) Erfinder:
- **Schuch, Dominik**
  **42781 Haan (DE)**
- **Berkels, Wolfgang**
  **46238 Bottrop (DE)**
- **Springer, Oliver**
  **46485 Wesel (DE)**
- **Hartung, Christian**
  **45133 Essen (DE)**
- **Condé, Hilke**
  **45966 Gladbeck (DE)**
- **Klann-Metz, Bärbel**
  **45141 Essen (DE)**

(56) Entgegenhaltungen:
**EP-A2- 1 197 559** **WO-A1-2004/041769**
**WO-A1-2007/027447** **US-A- 5 840 943**

- **R Wilson ET AL: "Research Section Overview of the Preparation, Use and Biological Studies on Polyglycerol Polyricinoleate (PGPR)", , 1. Januar 1998 (1998-01-01), XP055182499, Gefunden im Internet: URL:http://www.sciencedirect.com/science/article/pii/S027869159800057X/pdfft?md5=a95e0d68e434f70b0d3e033a1742dd2c&pid=1-s2.0-S027869159800057X-main.pdf [gefunden am 2015-04-13]**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

### Gebiet der Erfindung

Gegenstand der Erfindung sind Polyglycerinpartialester einer bestimmten Zusammensetzung, die in der Lage sind, sehr hydrophobe, öllösliche Substanzen in wässriger Lösung zu solubilisieren. Auch die Herstellung und Verwendung dieser polyglycoletherfreien Solubilisatoren in kosmetischen Formulierungen ist Bestandteil dieser Erfindung.

### Stand der Technik

Als Solubilisatoren öllöslicher, hydrophober Stoffe in Wasser werden zumeist nichtionische Tenside eingesetzt, die kaum schaumerzeugende Wirkung aufweisen.

Standardmäßig werden polyethoxylierte Triglyceride, insbesondere auf Basis von Rizinusöl, z.B. PEG-40 Hydrogenated Castor Oil, als Lösungsvermittler eingesetzt. Letzteres ist in der Lage, öllösliche Substanzen unterschiedlichster Struktur und Hydrophobizität in Wasser annähernd klar zu solubilisieren. Auch polyglycoletherfreie Polyglycerinpartialester werden seit einigen Jahren als alternative Solubilisatoren eingesetzt.

Der Nachteil aller bislang verfügbaren Polyglycerinpartialester ist, dass mit ihrer Verwendung als Solubilisatoren für öllösliche Substanzen in Wasser kein so breites Substanzspektrum abgedeckt werden kann wie mit den zuvor genannten polyethoxylierten Triglyceriden. So eigenen sich die Polyglycerinester hauptsächlich für die Solubilisierung "kleiner" Moleküle, wie z.B. kurzkettige Terpene. Fettsäurebasierte Öle sowie Triglyceride mit langkettigen Fettsäuren, wie z.B. Jojobaöl, Mandelöl, Sojaöl oder Avocadoöl lassen sich mit den auf Polyglycerinpartialestern-basierten Marktprodukten dagegen bislang noch nicht klar in Wasser solubilisieren.

JP 2008-119568 beschreibt die Verwendung von Polyglycerinpartialestern als Solubilisisatoren für Öle, wobei die Polyglycerinpartialester auf Mischungen von gesättigten Fettsäuren mit 8 bis 22 Kohlenstoffatomen und ungesättigten Fettsäuren mit 16 bis 22 Kohlenstoffatomen, wobei das molare Verhältnis von gesättigten zu ungesättigten Fettsäuren in einem Bereich von 0,2 zu 0,8 bis 0,8 zu 0,2 liegt, basieren. Zusätzlich können diesem Polyglycerinpartialester Polyglycerinpartialester der Polyrizinolsäure beigemischt werden. WO 2007/027447 offenbart Ester von Polyglycerin und Hydroxylgruppe enthaltender Fettsäuren. Aufgabe der vorliegenden Erfindung war es, polyglycerinpartialesterbasierte, Solubilisatoren zur Verfügung zu stellen, die im Gegensatz zu den bislang im Markt verfügbaren Produkten dazu in der Lage sind, besonders hydrophobe, öllösliche Substanzen, wie z.B. langkettige Triglyceride, klar in Wasser und kosmetische Formulierungen zu solubilisieren.

### Beschreibung der Erfindung

Überraschenderweise wurde gefunden, dass die im Folgenden beschriebenen Polyglycerinpartialester die Nachteile aus dem Stand der Technik beheben und damit die der Erfindung gestellte Aufgabe zu lösen vermögen.

Gegenstand der Erfindung sind daher Polyglycerinpartialester, die aus Polyglycerin durch Veresterung mit einem speziell ausgewählten Fettsäuregemisch hergestellt werden. Die Produkte sind in der Lage, sehr hydrophobe, öllösliche Substanzen, wie z.B. langkettige Triglyceride, klar in Wasser oder einer kosmetischen Formulierung zu solubilisieren. Auch die Herstellung und Verwendung dieser Solubilisatoren in kosmetischen Formulierungen ist Bestandteil dieser Erfindung.

Ein Vorteil der vorliegenden Erfindung ist, dass die hier beschriebenen Polyglycerinpartialester in der Lage sind, stark hydrophobe, öllösliche Substanzen, wie z.B. langkettige Triglyceride, klar in Wasser oder einer kosmetischen Formulierung zu solubilisieren, was mit den bisher verfügbaren Polyglycerinester-basierten Produkten nicht möglich ist.

Ein weiterer Vorteil ist, dass die hier beschrieben Polyglycerinpartialester im Gegensatz zu polyethoxylierten Triglyceriden aus ausschließlich nachwachsenden Rohstoffen hergestellt werden können.

Ein weiterer Vorteil ist, dass die hier beschriebenen Polyglycerinpartialester im Gegensatz zu polyethoxylierten Triglyceriden flüssig und somit gut verarbeitbar sind. Ein weiterer Vorteil gegenüber den polyethoxylierten Triglyceriden ist, dass die hier beschriebenen Polyglycerylester im Gegensatz zu polyethoxylierten Triglyceriden (PEG-40 Hydrogenated Castor Oil) zu augenscheinlich besonders klaren Dispersionen des Öls im Wasser führen und auch bei Lagerung keine Eintrübung erfolgt.

Noch ein Vorteil der vorliegenden Erfindung ist, dass man Formulierungen bereitstellen kann, die polyglycoletherfrei sind.

Ein weiterer Vorteil der hier beschriebenen Polyglycerinpartialester ist, dass diese ein angenehmes Hautgefühl in kosmetischen Formulierungen erzeugen können.

Noch ein Vorteil der hier beschriebenen Polyglycerinpartialester ist, dass diese in Wasser unter Rühren nur eine sehr geringe Schaumbildung zeigen.

Ein weiterer Vorteil ist, dass die hier beschrieben Polyglycerinpartialester eine nur sehr geringe Wirkung auf

Anschäumbarkeit und Schaummenge in Tensidformulierungen zeigen, dabei aber die Schaumcremigkeit verbessern können.

Noch ein Vorteil ist, dass die hier beschriebenen Polyglycerinpartialester in Tensidformulierungen zur Abmilderung der Reizwirkung auf die Haut führen können. Ein weiterer Vorteil der hier beschriebenen Polyglycerinpartialester ist, dass diese in Emulsionen eine stabilisierende Wirkung aufweisen können.

Ein weiterer Vorteil der erfindungsgemäßen Produkte ist, dass diese relativ stabil gegenüber Oxidation sind und stabiler bzgl. Farbe, Geruch und Aussehen.

Gegenstand der vorliegenden Erfindung sind Polyglycerinpartialester der allgemeinen Formel I

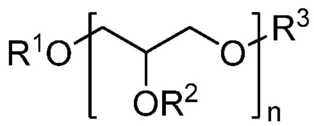

allgemeine Formel I

mit

- n = 2 bis 16, bevorzugt 4 - 14, besonders bevorzugt 5 - 11
- $R^1$, $R^2$, $R^3$ = unabhängig voneinander gleich oder verschieden ausgewählt aus H, $R^4$ und $R^5$, wobei
- $R^4$ = keine Hydroxylgruppe enthaltender, gesättigter oder ungesättigter Acylrest mit 6 - 22 C-Atomen, bevorzugt mit 8 - 18 C-Atomen,
- $R^5$ = mindestens eine Hydroxylgruppe enthaltender, gesättigter oder ungesättigter Acylrest mit 6 - 22 C-Atomen, bevorzugt mit 14 - 22 C-Atomen oder ein Acylrest eines Oligomers von mindestens eine Hydroxylgruppe enthaltenden, gesättigten oder ungesättigten Acylresten mit 6 - 22 C-Atomen, bevorzugt mit 14 - 22 C-Atomen, wobei der Acylrest des Oligomers bevorzugt 26 bis 66 C-Atome aufweist,
- dadurch gekennzeichnet, dass das Molverhältnis der Acylreste $R^4$ zu $R^5$ in einem Bereich von 95:5 bis 50:50 liegt.

Dem Fachmann ist bewusst, dass das in der allgemeinen Formel I enthaltene Polyglycerin-Grundgerüst aufgrund seiner polymeren Eigenschaft eine statistische Mischung verschiedener Verbindungen darstellt. Polyglycerin kann ausgebildete Etherbindungen zwischen zwei primären, einer primären und einer sekundären sowie zwei sekundären Positionen der Glycerinmonomere aufweisen. Aus diesem Grund besteht das Polyglycerin-Grundgerüst üblicherweise nicht ausschließlich aus linear verknüpften Glycerin-Einheiten, sondern kann auch Verzweigungen und Cyclen enthalten. Für Details siehe z.B. "Original synthesis of linear, branched and cyclic oligoglycerol Standards", Cassel et al., J. Org. Chem. 2001, 875-896.

Entsprechende Strukturen sind von der in dieser Hinsicht vereinfachten, allgemeinen Formel I miterfasst.

Durch den Begriff "das Molverhältnis der Acylreste $R^4$ zu $R^5$ liegt in einem Bereich von 95:5 bis 5:95" ist klar, dass im erfindungsgemäßen Polyglycerinpartialester Reste $R^4$ und $R^5$ enthalten sind.

Erfindungsgemäß bevorzugte Polyglycerinpartialester sind dadurch gekennzeichnet, dass sie Strukturen der allgemeinen Formel 1) enthalten, die jeweils mindestens einen Rest $R^4$ und einen Rest $R^5$ gleichzeitig aufweisen.

Die Acylreste $R^4$ und $R^5$ können statistisch an das Polyglyceringrundgerüst sowohl über primäre als auch über sekundäre Hydroxylgruppen gebunden sein.

Alle Bedingungen wie beispielsweise Druck und Temperatur sind, wenn nicht anders angegeben, Standardbedingungen (20 °C, 1 bar). Prozentzahlen sind, soweit nicht anders beschrieben, in Massenprozent angegeben.

Der Polymerisationsgrad n lässt sich dadurch bestimmen, dass die Hydroxylzahl des zur Synthese des erfindungsgemäßen Esters eingesetzten Polyglycerins ermittelt wird, wobei der mittlere Polymerisationsgrad n mit der Hydroxylzahl des zugrundeliegenden Polyglycerins über folgende Gleichung verknüpft ist:

$$n = \frac{\frac{2000 \bullet M(KOH)}{OHZ} - M(Wasser)}{\left[ \left[ M(Glycerin) - M(Wasser) \right] - \frac{1000 \bullet M(KOH)}{OHZ} \right]}$$

mit M = molare Masse; OHZ = Hydroxylzahl des freien Polyglycerins.

Alternativ lässt sich der Polymerisationsgrad n auch dadurch bestimmen, dass die Hydroxylzahl des nach vollständiger Esterhydrolyse erhaltenen Polyglycerins ermittelt wird.

Geeignete Bestimmungsmethoden zur Ermittlung der Hydroxylzahl sind insbesondere solche gemäß DGF C-V 17 a (53), Ph. Eur. 2.5.3 Method A und DIN 53240.

Die Acylreste $R^4$ und $R^5$ sind bevorzugt Acylreste von Fettsäuren. $R^4$ und $R^5$ können auch Mischungen solcher Acylreste repräsentieren, insbesondere technische Mischungen wie beispielsweise im Falle von $R^4$ Kokosfettsäureschnitte.

Für $R^4$ ist es insbesondere bevorzugt, dass bezogen auf alle Reste $R^4$ im Polyglycerinpartialester mindestens 50 Mol-%, bevorzugt mindestens 75 Mol-% der Acylreste $R^4$ ausgewählt sind aus Capryloyl-, Caproyl- und Lauroylresten.

Für $R^5$ ist es insbesondere bevorzugt, dass er ausgewählt ist aus Ricinoyl- und Hydroxystearoylresten, ihren Oligomeren und Mischungen davon, insbesondere bevorzugt bestehen bezogen auf alle Reste $R^5$ im Polyglycerinpartialester mindestens 90 Mol-% der Acylreste $R^5$ aus Ricinoylresten oder einer Mischung von Ricinoyl- und Hydroxystearoylresten, wobei es bevorzugt ist, dass die Mischung von Ricinoyl- und Hydroxystearoylresten ein Molverhältnis von Ricinoyl- zu Hydroxystearoylresten in einem Bereich von 100 zu 0,1 bis 50 zu 50 aufweist.

Alternativ bevorzugt ist $R^5$ ausgewählt aus Ricinoylresten.

Erfindungsgemäß bevorzugte Polyglycerinpartialester sind dadurch gekennzeichnet, dass das Gewichtsverhältnis des Polyglycerylrests zur Summe der Acylreste $R^4$ und $R^5$ 85:15 bis 55:45, bevorzugt 80:20 bis 60:40, besonders bevorzugt 75:25 bis 65:35 beträgt.

Erfindungsgemäß bevorzugte Polyglycerinpartialester sind weiterhin dadurch gekennzeichnet, dass das Molverhältnis von gesättigten zu ungesättigten Acylresten in Summe aller Reste $R^4$ und $R^5$ 99:1 - 1:99, bevorzugt 95:5 - 50:50, besonders bevorzugt 90:10 - 60:40 beträgt.

Die Polyglycerinpartialester der vorliegenden Erfindung lassen sich durch klassische Veresterungs- und Umesterungsverfahren herstellen, bevorzugt mit dem im Folgenden beschriebenen erfindungsgemäßen Verfahren.

Ein weiterer Gegenstand der vorliegenden Erfindung ist ein Verfahren zur Herstellung von Polyglycerinpartialestern umfassend die Verfahrensschritte:

A) Bereitstellen eines Polyglycerins mit einem mittleren Polymerisationsgrad n = 2 bis 16, bevorzugt 4 - 14, besonders bevorzugt 5 - 11,

B) Acylierung eines Teils der Hydroxygruppen des Polyglycerins mit

mindestens einem ersten Carbonsäurederivat einer oder mehrerer ersten, gesättigten oder ungesättigten, keine Hydroxygruppe enthaltenden Carbonsäuren mit 6 - 22 C-Atomen, bevorzugt mit 8 - 18 C-Atomen und

mindestens einem zweiten Carbonsäurederivat einer oder mehrerer zweiten, gesättigten oder ungesättigten, mindestens eine Hydroxygruppe enthaltenden Carbonsäuren mit 6 - 22 C-Atomen, bevorzugt mit 14 - 22 C-Atomen, oder eines Oligomers der zweiten Carbonsäure, wobei das Oligomer bevorzugt 26 bis 66 C-Atome aufweist,

wobei die Carbonsäurederivate ausgewählt sind aus Carbonsäuren und Carbonsäureestern, wobei als Carbonsäureester Triglyceride erfindungsgemäß bevorzugt sind,

wobei das molare Verhältnis der Acylreste des in Verfahrensschritt B) eingesetzten ersten Carbonsäurederivats zu denen des zweiten Carbonsäurederivats in einem Bereich von 95:5 bis 5:95, bevorzugt von 85:5 bis 15:85, besonders bevorzugt 85:15 bis 50:50 liegt.

Die in dem erfindungsgemäßen Verfahren bevorzugt eingesetzten Carbonsäurederivate sind Fettsäurederivate.

In dem erfindungsgemäßen Verfahren können somit in Verfahrensschritt B)

mindestens eine erste Carbonsäure und mindestens eine zweite Carbonsäure,

mindestens ein erster Carbonsäureester und mindestens eine zweite Carbonsäure,

mindestens eine erste Carbonsäure und mindestens ein zweiter Carbonsäureester, mindestens ein erster Carbonsäureester und mindestens ein zweiter Carbonsäureester,

mindestens eine erste Carbonsäure und mindestens ein erster Carbonsäureester und mindestens eine zweite Carbonsäure,

mindestens eine erste Carbonsäure und mindestens ein erster Carbonsäureester und mindestens ein zweiter Carbonsäureester,

mindestens eine erste Carbonsäure und mindestens eine zweite Carbonsäure und mindestens ein zweiter Carbonsäureester,

mindestens ein erster Carbonsäureester und mindestens eine zweite Carbonsäure und mindestens ein zweiter Carbonsäureester

oder

mindestens eine erste Carbonsäure und mindestens ein erster Carbonsäureester und mindestens eine zweite Carbonsäure und mindestens ein zweiter Carbonsäureester, eingesetzt werden.

Ein erfindungsgemäß bevorzugtes Verfahren ist dadurch gekennzeichnet, dass bezogen auf die Acylreste aller ersten Carbonsäurederivate mindestens 50 Mol-%, bevorzugt mindestens 75 Mol-% der ersten Carbonsäuren ausgewählt sind aus Caprylsäure, Caprinsäure und Laurinsäure.

Es ist erfindungsgemäß bevorzugt, dass in dem erfindungsgemäßen Verfahren bezogen auf die Acylreste aller zweiten Carbonsäurederivate mindestens 90 Mol-% der zweiten Carbonsäuren ausgewählt sind aus Rizinolsäure und Hydroxystearinsäure.

Ein erfindungsgemäß bevorzugtes Verfahren ist dadurch gekennzeichnet, dass bezogen auf die Acylreste aller zweiten Carbonsäurederivate mindestens 90 Mol-% der zweiten Carbonsäuren aus Rizinolsäure und/oder Hydroxystearinsäure bestehen, wobei es bevorzugt ist, dass die zweiten Carbonsäuren ein Molverhältnis von Rizinolsäureresten zu Hydroxystearinsäureresten in einem Bereich von 100 zu 0,1 bis 50 zu 50 aufweisen.

Alternativ bevorzugt ist das zweite Carbonsäurederivat ausgewählt aus Rizinolsäure oder Rizinusöl.

Es ist erfindungsgemäß bevorzugt, dass in dem erfindungsgemäßen Verfahren das Gewichtsverhältnis des Polyglycerins zur Summe der Acylreste der berechnet eingesetzten ersten und zweiten Carbonsäurederivate 85:15 bis 55:45, bevorzugt 80:20 bis 60:40, besonders bevorzugt 75:25 bis 65:35 beträgt.

Ein erfindungsgemäß bevorzugtes Verfahren ist dadurch gekennzeichnet, dass das Molverhältnis der Acylreste von gesättigten zu denen der ungesättigten Carbonsäurederivate, die in Verfahrensschritt B) eingesetzt werden, 99:1 - 1:99, bevorzugt 95:5 - 50:50, besonders bevorzugt 90:10 - 60:40 beträgt.

Ein weiterer Gegenstand der vorliegenden Erfindung sind Polyglycerinpartialester, erhältlich nach dem erfindungsgemäßen Verfahren, wobei erfindungsgemäß solche Partialester bevorzugt sind, die mit erfindungsgemäß bevorzugten Verfahren erhältlich sind.

Ein weiterer Gegenstand der vorliegenden Erfindung sind Formulierungen, insbesondere kosmetische und pharmazeutische, wobei kosmetische besonders bevorzugt sind, die mindestens einen erfindungsgemäßen Polyglycerinpartialester und/oder mindestens einen Polyglycerinpartialester erhältlich nach dem erfindungsgemäßen Verfahren enthalten.

Insbesondere sind Formulierungen bevorzugt, die im Wesentlichen polyglycoletherfrei und im Wesentlichen frei von alkoxylierten Verbindungen sind. Unter dem Begriff "im Wesentlichen frei von alkoxylierten Verbindungen" und "im Wesentlichen polyglycoletherfrei" im Zusammenhang mit der vorliegenden Erfindung ist zu verstehen, dass die Formulierungen keine nennenswerten Mengen an alkoxylierten oder polyglycolether enthaltende Verbindungen aufweist, die eine oberflächenaktive Wirkung ausüben. Insbesondere ist hierunter zu verstehen, dass diese Verbindungen in Mengen von kleiner 1 Gew.-%, bevorzugt von kleiner 0,1 Gew.-%, besonders bevorzugt von kleiner 0,01 Gew.-% bezogen auf die Gesamtformulierung, insbesondere keine nachweisbaren Mengen, enthalten sind.

Die Polyglycerinpartialester der vorliegenden Erfindung lassen sich vorteilhaft zur Herstellung von Pflege- und Reinigungsformulierungen, insbesondere für Haut und Hautanhangsgebilden, wie beispielsweise Flüssigseifen, Duschgels, Ölbädern, Make-up Removern oder Shampoos, Duschgels, Schaumbäder, Flüssigseifen, Haarshampoos, 2in1-Shampoos, Haarspülungen, Dauerwell-Fixierlösungen, Haarfärbeshampoos, Haarfestiger, Haarkuren, Haarlegemittel, Haarstyling-Zubereitungen, Fön-Lotionen, Schaumfestiger, Haarkuren, Leave-In Conditionierer, Haarglättungsmitteln, Glanzverbesserungsmitteln und Mittel zum Färben der Haare verwenden. Somit sind derartige Verwendungen ebenfalls Gegenstand der vorliegenden Erfindung.

Noch ein Gegenstand der vorliegenden Erfindung sind somit auch Pflege- und Reinigungsformulierungen, insbesondere für Haut und Hautanhangsgebilden, enthaltend erfindungsgemäße Polyglycerinpartialester.

Unter dem Begriff "Pflegeformulierung" wird hier eine Formulierung verstanden, die den Zweck erfüllt, einen Gegenstand in seiner ursprünglichen Form zu erhalten, die Auswirkungen äußerer Einflüsse (z.B. Zeit, Licht, Temperatur, Druck, Verschmutzung, chemische Reaktion mit anderen, mit dem Gegenstand in Kontakt tretenden reaktiven Verbindungen) wie beispielsweise Altern, Verschmutzen, Materialermüdung, Ausbleichen, zu mindern oder zu vermeiden oder sogar gewünschte positive Eigenschaften des Gegenstandes zu verbessern. Für letzten Punkt sei etwa ein Glanz des betrachteten Gegenstandes genannt.

Erfindungsgemäße kosmetische Pflege- und Reinigungsformulierungen, können zum Beispiel mindestens eine zusätzliche Komponente enthalten, ausgewählt aus der Gruppe der

Emollients,
Emulgatoren,
Verdicker/Viskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,

Farbstoffe,
kosmetische Wirkstoffe,
Pflegeadditive,
Überfettungsmittel,
Lösungsmittel.

Substanzen, die als beispielhafte Vertreter der einzelnen Gruppen eingesetzt werden können, sind dem Fachmann bekannt und können beispielsweise der EP2273966A1 entnommen werden. Diese Patentanmeldung wird hiermit als Referenz eingeführt und gilt somit als Teil der Offenbarung.

Bezüglich weiterer fakultativer Komponenten sowie der eingesetzten Mengen dieser Komponenten wird ausdrücklich auf die dem Fachmann bekannten einschlägigen Handbücher, zum Beispiel K. Schrader, "Grundlagen und Rezepturen der Kosmetika", 2. Auflage, Seite 329 bis 341, Hüthig Buch Verlag Heidelberg, verwiesen.

Die Mengen der jeweiligen Zusätze richten sich nach der beabsichtigten Verwendung. Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Bevorzugte erfindungsgemäße Formulierung enthalten oben als erfindungsgemäß bevorzugt herausgestellte Polyglycerinpartialester bzw. oben als erfindungsgemäß bevorzugt herausgestellte Polyglycerinpartialester erhältlich nach dem erfindungsgemäßen Verfahren.

Es ist bevorzugt, wenn die erfindungsgemäße Formulierung zusätzlich mindestens eine öllösliche Substanz und Wasser enthalten.

Als öllösliche Substanzen werden in der vorliegenden Erfindung Substanzen verstanden, die einen LogP-Wert (Logarithmus von n-Ocantol-Wasser-Verteilungskoefizient, auch log $K_{OW}$ genannt) von mindestens 2 aufweisen.

Bevorzugte öllösliche Substanzen weisen einen LogP-Wert von mindestens 5 auf.

Besonders bevorzugte öllösliche Substanzen sind ausgewählt aus der Gruppe umfassend fettsäurebasierte Öle, Triglyceride mit langkettigen Fettsäuren, kosmetische Esteröle, reine Kohlenwasserstoffe wie z.B. Jojobaöl, Mandelöl, Sojaöl, Avocadoöl, Olivenöl, Arganöl, Rapsöl, Sonnenblumenöl, Neemöl, Capryl-/Caprinsäuretriglycerid, Sheabutter, Decylcocoat, Isopropylpalmitat, Myristylmyristat und Isohexadecan.

Besonders bevorzugte erfindungsgemäße Formulierungen enthalten
0,1 Gew.-% bis 40 Gew.-%, bevorzugt 0,3 Gew.-% bis 35 Gew.-%, besonders bevorzugt 0,5 Gew.-% bis 10 Gew.-%, erfindungsgemäßen Polyglycerinpartialester und/oder Polyglycerinpartialester erhältlich nach dem erfindungsgemäßen Verfahren, 0,01 Gew.-% bis 40 Gew.-%, bevorzugt 0,1 Gew.-% bis 30 Gew.-%, besonders bevorzugt 0,2 Gew.-% bis 2 Gew.-%, öllösliche Substanz und
10 Gew.-% bis 98 Gew.-%, bevorzugt 20 Gew.-%bis 95 Gew.-%, besonders bevorzugt 45 Gew.-% bis 90 Gew.-% Wasser.

Noch ein Gegenstand der vorliegenden Erfindung ist die Verwendung mindestens eines erfindungsgemäßen Polyglycerinpartialesters und/oder mindestens eines Polyglycerinpartialesters erhältlich nach dem erfindungsgemäßen Verfahren zur Solubilisierung von mindestens einer öllöslichen Substanz in Wasser, wobei es erfindungsgemäß bevorzugt ist, oben als erfindungsgemäß bevorzugt herausgestellte Polyglycerinpartialester bzw. oben als erfindungsgemäß bevorzugt herausgestellte Polyglycerinpartialester erhältlich nach dem erfindungsgemäßen Verfahren zu verwenden.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Beispiele:

*Beispiel 1: Herstellung erfindungsgemäßer Polyglycerinpartialester*

1.1. Herstellung des Polyglycerinpartialesters A:

Unter Stickstoffatmosphäre wurden 225 g Polyglycerin (Hydroxylzahl = 935 mg KOH / g) mit 39,4 g Capryl/Caprinsäure und 20,8 g raffinierter Kokosfettsäure und 22,1 g Rizinolsäure und 15,5 g Hydroxystearinsäure bei 240 °C gerührt bis eine Säurezahl von < 0,5 mg KOH / g erreicht war. Das im Verlauf der Reaktion gebildete Wasser wurde kontinuierlich abdestilliert. Nach Abkühlung auf Raumtemperatur lag das Reaktionsprodukt als trübe Flüssigkeit vor.

1.2. Herstellung des Polyglycerinpartialesters B:

Unter Stickstoffatmosphäre wurden 225 g Polyglycerin (Hydroxylzahl = 996 mg KOH / g) mit 39,4 g Capryl/Caprinsäure und 20,8 g raffinierter Kokosfettsäure und 22,1 g Rizinolsäure und 15,5 g Hydroxystearinsäure bei 240 °C

gerührt bis eine Säurezahl von < 0,5 mg KOH / g erreicht war. Das im Verlauf der Reaktion gebildete Wasser wurde kontinuierlich abdestilliert. Nach Abkühlung auf Raumtemperatur lag das Reaktionsprodukt als trübe Flüssigkeit vor.

1.3. Herstellung des Polyglycerinpartialesters C:

Unter Stickstoffatmosphäre wurden 225 g Polyglycerin (Hydroxylzahl = 935 mg KOH / g) mit 47,3 g Capryl/Caprinsäure und 20,8 g raffinierter Kokosfettsäure und 17,7 g Rizinolsäure und 12,4 g Hydroxystearinsäure bei 240 °C gerührt bis eine Säurezahl von < 0,5 mg KOH / g erreicht war. Das im Verlauf der Reaktion gebildete Wasser wurde kontinuierlich abdestilliert. Nach Abkühlung auf Raumtemperatur lag das Reaktionsprodukt als trübe Flüssigkeit vor.

1.4. Herstellung des Polyglycerinpartialesters D:

Unter Stickstoffatmosphäre wurden 202 g Polyglycerin (Hydroxylzahl = 884 mg KOH / g) mit 37,7 g Capryl/Caprinsäure und 24,9 g raffinierter Kokosfettsäure und 23,5 g Rizinolsäure und 12,4 g Hydroxystearinsäure bei 240 °C gerührt bis eine Säurezahl von < 0,5 mg KOH / g erreicht war. Das im Verlauf der Reaktion gebildete Wasser wurde kontinuierlich abdestilliert. Nach Abkühlung auf Raumtemperatur lag das Reaktionsprodukt als trübe Flüssigkeit vor.

1.5. Herstellung des Polyglycerinpartialesters E:

Unter Stickstoffatmosphäre wurden 225 g Polyglycerin (Hydroxylzahl = 935 mg KOH / g) mit 39,4 g Capryl/Caprinsäure und 23,0 g raffiniertem Kokosöl und 41,5 g Rizinusöl bei 240 °C gerührt bis eine Säurezahl von < 0,5 mg KOH / g erreicht war. Das im Verlauf der Reaktion gebildete Wasser wurde kontinuierlich abdestilliert. Nach Abkühlung auf Raumtemperatur lag das Reaktionsprodukt als trübe Flüssigkeit vor.

1.6. Herstellung des Polyglycerinpartialesters F:

Unter Stickstoffatmosphäre wurden 225 g Polyglycerin (Hydroxylzahl = 935 mg KOH / g) mit 39,4 g Capryl/Caprinsäure und 23,0 g raffiniertem Kokosöl und 23,0 g Rizinusöl und 14,2 g Castorwachs bei 240 °C gerührt bis eine Säurezahl von < 0,5 mg KOH / g erreicht war. Das im Verlauf der Reaktion gebildete Wasser wurde kontinuierlich abdestilliert. Nach Abkühlung auf Raumtemperatur lag das Reaktionsprodukt als trübe Flüssigkeit vor.

1.7. Herstellung des Polyglycerinpartialesters G:

In einer ersten Reaktion wurden 225 g Polyglycerin (Hydroxylzahl = 935 mg KOH / g) unter Stickstoffatmosphäre mit 38,0 g Caprylsäure und 60 g Rizinusöl bei 240 °C gerührt bis eine Säurezahl von < 0,5 mg KOH / g erreicht war. Das im Verlauf der Reaktion gebildete Wasser wurde kontinuierlich abdestilliert.
In einer zweiten Reaktion wurden 225 g Polyglycerin (Hydroxylzahl = 1060 mg KOH / g) unter identischen Reaktionsbedingungen mit 41,0 g Caprinsäure, 23 g Rizinusöl und 46,0 g raffiniertem Kokosöl umgesetzt.
Anschließend wurden die zwei Produkte vereinigt und bei 90 °C gerührt bis eine klare, homogene Mischung entstanden war, die nach Abkühlung auf Raumtemperatur als trübe Flüssigkeit vorlag.

*Beispiel 2: Herstellung nicht-erfindungsgemäßer Polyglycerinpartialester*

2.1. Herstellung des Polyglycerinpartialesters H:

Unter Stickstoffatmosphäre wurden 394 g Polyglycerin (Hydroxylzahl = 1061 mg KOH / g) mit 51,3 g Capryl/Caprinsäure und 23,0 g raffinierter Kokosfettsäure und 32,8 g Ölsäure bei 240 °C gerührt bis eine Säurezahl von < 0,5 mg KOH / g erreicht war. Das im Verlauf der Reaktion gebildete Wasser wurde kontinuierlich abdestilliert. Nach Abkühlung auf Raumtemperatur lag das Reaktionsprodukt als trübe Flüssigkeit vor.

*Beispiel 3: Nicht-erfindungsgemäße,kommerzielle Vergleichsbeispiele*

3.1. TEGOSOFT® PC 41:

Standardsolubilisator, polyetherfrei. INCI: Polyglyceryl-4 Caprate. Verkaufsprodukt der Evonik Industries AG.

3.2. NATRAGEM® S 150 NP-LQ-(CM):

Solubilisator für Öle, polyetherfrei. INCI: Polyglyceryl-4 Laurate/Sebacate (and) Polyglyceryl-4 Caprylate/Caprate (and) Water. Verkaufsprodukt von Croda.

3.3. TAGAT® CH 40:

Standardsolubilisator, polyetherhaltig. INCI: PEG-40 Hydrogenated Castor Oil. Verkaufsprodukt der Evonik Industries AG.

Im Folgenden wurden die oben beschriebenen Produkte in kosmetischen Formulierungen ausgetestet.

Die Formulierungsbestandteile sind in den Zusammensetzungen in Form der allgemein anerkannten INCI-Nomenklatur unter Verwendung der englischen Begriffe benannt. Alle Konzentrationen in den Anwendungsbeispielen sind in Gewichtsprozent angegeben.

*Beispiel 4: Verbessertes Lösungsvermögen der erfindungsgemäßen Polyglycerinpartialestern im Vergleich zu den nicht-erfindungsgemäßen Polyglycerinpartialestern in wässrigen Lösungen*

Um das Lösungsvermögen der erfindungsgemäßen Polyglycerinpartialestern zu untersuchen, wurden diese mit kosmetischen Ölen gemischt und mit Wasser versetzt. Als Öle wurden Avocadoöl (Lieferant: Gustav Heess) und Caprylic/Capric Triglyceride (TEGOSOFT® CT, Evonik Industries AG) getestet. Es wurde untersucht, welcher Anteil an Solubilisator nötig ist, um 0,5% des jeweiligen Öls vollkommen klar in Wasser zu lösen. Dazu wurde der Solubilisator (unterschiedliche Mengen) mit dem Öl (0,5 g) gut gemischt und dann unter Rühren langsam mit Wasser (auf 100 g aufgefüllt) versetzt. Es wurde eine Stunde bei 45 °C gerührt. Ein "klares Gemisch" darf nach Abkühlung auf 20 °C innerhalb von 1 Woche nicht wieder trüb werden.

In Tabelle 1 sind die erhaltenen Massenverhältnisse von Solubilisator zu Öl zusammengefasst, die nötig waren, klare Mischungen zu erhalten.

Tabelle 1: Solubilisator-zu-Öl Verhältnis, das für eine klare Lösung des Öls in Wasser nötig war

| | Avocadoöl | Caprylic/Capric Triglyceride |
|---|---|---|
| Polyglycerinpartialester A | 10:1 | 4:1 |
| Polyglycerinpartialester E | 8:1 | 4:1 |
| Polyglycerinpartialester F | 9:1 | 4:1 |
| Polyglycerinpartialester G | 11:1 | 5:1 |
| Polyglycerinpartialester H (nicht erfindungsgemäß) | >20:1 | 9:1 |
| TEGOSOFT® PC 41 (nicht erfindungsgemäß) | >20:1 | 16:1 |
| TAGAT® CH 40 (nicht erfindungsgemäß) | 6:1 | 6:1 |

Anhand der Ergebnisse in Tabelle 1 ist ersichtlich, dass die erfindungsgemäßen Polyglycerinpartialester A, E, F und G deutlich bessere Solubilisierungseigenschaften aufweisen als die polyetherfreien Vergleichsbeispiele Polyglycerinpartialester H und TEGOSOFT® PC 41. Überraschenderweise werden sogar ähnliche Solubilisator-zu-Öl Verhältnisse erreicht wie für den polyetherhaltigen Standardsolubilisator TAGAT® CH 40 und dessen Performance sogar teilweise übertroffen.

*Beispiel 5: Verbessertes Lösungsvermögen der erfindungsgemäßen Polyglycerinpartialestern im Vergleich zu den nicht-erfindungsgemäßen Polyglycerinpartialestern in tensidischen Formulierungen*

Neben den in Beispiel 4 gezeigten Lösungseigenschaften der erfindungsgemäßen Polyglycerinpartialester für Öle in Wasser wurde das Lösungsvermögen für Öle auch in tensidischen Formulierungen untersucht.

Dazu wurde der jeweilige Solubilisator mit 0,5 g Caprylic/Capric Triglyceride (TEGOSOFT® CT, Evonik Industries AG) bei 60 °C für 5 min gemischt. Dann wurde langsam das Wasser bei 60 °C unter Rühren hinzugegeben und 10 min gerührt. Das Gemisch wurde dann innerhalb von 30 min auf 30 °C abgekühlt. Unter Rühren wurde die Mischung daraufhin mit den Tensiden versetzt.

Es wurde untersucht, welcher Anteil an Solubilisator nötig ist, um 0,5% des Öls vollkommen klar in dem jeweiligen Tensidsystem zu lösen. Zwei Tensidsysteme wurden verwendet: eine Standard Laurylethersulfat/Betain-Mischung (Tabelle 2) und eine polyetherfreie Formulierung (Tabelle 3). In Tabelle 4 sind die erhaltenen Massenverhältnisse von Solubilisator-zu-Öl zusammengefasst, die nötig waren, um klare Mischungen zu erhalten.

Tabelle 2: Formulierung Y zur Beurteilung der Solubilisierungseigenschaften in einem Standardtensidsystem

| Solubilisator | X% |
|---|---|
| TEGOSOFT® CT, Evonik Industries AG, (INCI: Caprylic/Capric Triglyceride) | 0,5% |
| Wasser | ad 100,0% |
| Texapon® NSO, BASF Cognis, 28%-ig, (INCI: Sodium Laureth Sulfate) | 32,0% |
| TEGO® Betain F 50, Evonik Industries AG, 38%-ig, (INCI: Cocamidopropyl Betaine) | 8,0% |
| Polymer JR 400, Amerchol, (Polyquaternium-10) | 0,2% |
| Citric Acid, 30% | ad pH 5,5 |

Tabelle 3: Formulierung Z zur Beurteilung der Solubilisierungseigenschaften in einem polyetherfreien Tensidsystem

| Solubilisator | X% |
|---|---|
| TEGOSOFT® CT, Evonik Industries AG, (INCI: Caprylic/Capric Triglyceride) | 0,5% |
| Wasser | ad 100,0% |
| REWOTERIC® AM C, Evonik Industries AG, 32%-ig, (INCI: Sodium Cocoamphoacetate) | 17,5% |
| Plantacare 1200 UP, BASF Cognis, 50%-ig, (INCI: Lauryl Glucoside) | 8,8% |
| Plantacare 818 UP, BASF Cognis, 51%-ig, (INCI: Coco-Glucoside) | 2,4% |
| PERLASTAN® SC 25 NKW, Schill&Seilacher, 25%-ig, (Disodium/Sodium Cocoyl Glutamate) | 14,4% |
| Citric Acid, 30% | ad pH 5,2 |

Tabelle 4: Solubilisator-zu-Öl Verhältnis, das für eine klare Lösung des TEGOSOFT® CT in den tensidischen Formulierungen nötig ist

| | TEGOSOFT® CT in Formulierung Y | TEGOSOFT® CT in Formulierung Z |
|---|---|---|
| Polyglycerinpartialester A | 6:1 | 8:1 |
| Polyglycerinpartialester F | 5:1 | 6:1 |
| NATRAGEM® S 150 NP-LQ-(CM) (nicht erfindungsgemäß) | 12:1 | 11:1 |
| TEGOSOFT® PC 41 (nicht erfindungsgemäß) | 12:1 | 14:1 |
| TAGAT® CH 40 (nicht erfindungsgemäß) | 8:1 | 5:1 |

Die Ergebnisse in Tabelle 4 zeigen, dass die erfindungsgemäßen Polyglycerinpartialester A und F deutlich verbesserte Lösungsvermittlereigenschaften aufweisen im Vergleich zu den Vergleichsprodukten NATRAGEM® S 150 NP-LQ-(CM) und TEGOSOFT® PC 41. Überraschenderweise wurden mit den erfindungsgemäßen Polyglycerinpartialester A und F teilweise auch bessere Ergebnisse erhalten als mit dem polyetherhaltigen Produkt TAGAT® CH 40.

*Beispiel 6: Verbessertes Hautpflegevermögen und Schaumeigenschaften der erfindungsgemäßen Polyglycerinpartialestern im Vergleich zu nicht-erfindungsgemäßen Polyglycerinpartialestern in Tensidmischungen*

Zur Bewertung der Hautpflegeleistung und der Schaumeigenschaften des erfindungsgemäßen Polyglycerinpartialesters G in wässrigen, tensidischen Formulierungen wurde ein sensorischer Handwaschtest im Vergleich zum Vergleichsbeispiel TEGOSOFT® PC 41 nach dem Stand der Technik durchgeführt.

Eine Gruppe bestehend aus 10 trainierten Prüfpersonen wusch sich dabei definiert die Hände und bewertete Schaumeigenschaften und Hautgefühl anhand einer Notenskala von 1 (schlecht) bis 5 (sehr gut).

Die Produkte wurden jeweils in einer standardisierten Tensidformulierung getestet, mit dem Standard-Tensidsystem 9% aktiv Sodium Laureth Sulfate und 3% aktiv Cocamidopropyl Betaine (Tabelle 5).

Tab. 5: Testformulierungen für den Handwaschtest:

| **Formulierungsbeispiele** | **U** | **V** | **W** |
|---|---|---|---|
| Texapon® NSO-IS, BASF Cognis, 28%-ig, (INCI: Sodium Laureth Sulfate) | 32,0% | 32,0% | 32,0% |
| TEGO® Betain F 50, Evonik Industries AG, 38%-ig, (INCI: Cocamidopropyl Betaine) | 8,0% | 8,0% | 8,0% |
| NaCl | 1,5% | 1,5% | 1,5% |
| Zitronensäure | 0,2% | 0,2% | 0,2% |
| Wasser, demineralisiert | 58,3% | 55,3% | 55,3% |
| Polyglycerinpartialester G (erfindungsgemäß) | - | 3,0% | - |
| TEGOSOFT® PC 41 (nicht erfindungsgemäß) | - | - | 3,0% |

Die sensorischen Testergebnisse sind in Tabelle 6 zusammengefasst.

Tab. 6: Ergebnisse des Handwaschtests:

| **Testformulierung** | **U** | **V** | **W** |
|---|---|---|---|
| Anschäumverhalten | 3,3 | 3,6 | 3,5 |
| Schaumvolumen | 3,1 | 3,3 | 3,2 |
| Schaumcremigkeit | 2,9 | **4,1** | 3,7 |
| Hautgefühl während des Waschens | 2,9 | 3,1 | 3,0 |
| Abwaschbarkeit | 3,3 | 3,8 | 3,7 |
| Hautglätte | 2,1 | **2,8** | 2,4 |
| Hautweichheit | 2,3 | **3,1** | 2,8 |
| Hautglätte nach 3 min. | 3,1 | **3,8** | 3,6 |
| Hautweichheit nach 3 min. | 2,9 | **3,7** | 3,4 |

Anhand der Testergebnisse in Tabelle 6 wird ersichtlich, dass die erfindungsgemäße Formulierung V unter Verwendung des erfindungsgemäßen Polyglycerinpartialesters G überraschenderweise in allen Applikationseigenschaften im Vergleich zur Vergleichsformulierung W nach dem Stand der Technik überlegen ist. Vor diesem Hintergrund sind die Ergebnisse der erfindungsgemäßen Formulierung V als sehr gut zu bezeichnen und zeigen eine deutliche Verbesserung gegenüber dem Stand der Technik.

Anhand der Messwerte ist ersichtlich, dass der erfindungsgemäße Polyglycerinpartialester G in der Formulierung V vor allem zu einer signifikanten Verbesserung speziell bei Schaumcremigkeit sowie bei der Hautglätte und der Hautweichheit führte.

*Weitere Formulierungsbeispiele:*

Die in den nachfolgenden Tabellen angegebenen Formulierungsbeispiele zeigen exemplarische Vertreter einer Vielzahl von möglichen erfindungsgemäßen Zusammensetzungen.

Falls die Herstellung der Formulierung zuvor die getrennte Zubereitung bzw. Mischung von Formulierungsbestandteilen erfordert, wird dieses als mehrphasige Zubereitung bezeichnet. Falls eine zweiphasige Herstellung erforderlich ist, werden die beiden Phasen mit A und B in den angegebenen Tabellen gekennzeichnet. Bei drei- bzw. mehrphasigen Prozessen werden die Phasen mit A, B und C etc. benannt. Wenn nicht anders angegeben handelt es sich bei den Angaben in den Tabellen um Angaben in Gew.-%. In den folgenden Formulierungsbeispielen sind die Angaben bzw. Gew.-% auf den jeweiligen Aktivstoff bezogen. Einige Produkte sind jedoch kommerziell als Lösungen in v.a. Wasser erhältlich, so dass in diesen Fällen dementsprechend je nach Aktivgehalt mehr von den kommerziellen Produkten eingesetzt wurde.

"Produkt Beispiel A bis G" entsprechen den "Polyglycerinpartialestern A bis G des Beispiels 1".

Tab. 7: Formulierung für Wet Wipes

| | |
|---|---|
| Butylene glycol | 2,0% |
| Glycerin | 1,0% |
| Produkt Beispiel A | 1,0% |
| Silicone Quaternium-22; Polyglycerin-3 Caprate; Dipropylene Glycol; Cocamidopropyl Betaine | 0,5% |
| Allantoin | 0,2% |
| Maltodextrin | 0,5% |
| Chamomilla extract | 0,1% |
| Phenoxyethanol; Ethylhexyl Glycerin | 0,7% |
| Perfume | q.s. |
| Water | ad 100,0% |
| Citric Acid, 30% | ad pH 5,5 |

Tab. 8: Creme-Bad

| | |
|---|---|
| Water | ad 100,0% |
| Sodium Laureth Sulfate | 8,0% |
| Coco-Glucoside | 4,0% |
| Cocamidopropyl Betaine | 5,0% |
| Produkt Beispiel B | 1,0% |
| PEG-18 Glyceryl Oleate/Cocoate | 2,0% |
| PEG-40 Sorbitan Peroleate | 1,4% |
| Perfume (fragrance) | 0,2% |
| Argania Spinosa Kernel Oil | 0,2% |
| Citrus Aurantifolia (Lime) Oil | 0,2% |
| Linalool | 0,1% |
| Coumarin | 0,1% |
| Glycerin | 0,5% |
| Glycol Distearate | 0,5% |
| Styrene/Acrylates Copolymer | 0,2% |
| Tocopherol | 0,1% |
| Preservative | q.s. |
| Citric Acid | ad pH 5,2 |

Tab. 9: Creme-Dusche

| | |
|---|---|
| Water | ad 100,0% |
| Glycerin | 4,0% |
| Sodium Laureth Sulfate | 4,0% |
| Cocamidopropyl Betaine | 3,5% |
| Produkt Beispiel G | 2,0% |
| Coco-Glucoside | 2,0% |
| Ricinus Communis Seed Oil (Seed) | 0,5% |
| Glyceryl Oleate | 0,5% |
| Argania Spinosa Kernel Oil | 0,1% |
| Butyrospermum Parkii Butter Extract | 0,1% |
| Limonene | 0,1% |
| Perfume (fragrance) | 0,2% |
| Acrylates/C10-30 Alkyl Acrylate Crosspolymer | 0,2% |
| Hydroxypropyl Methylcellulose | 0,2% |
| Styrene/Acrylates Copolymer | 0,2% |
| Sodium Hydroxide | 0,2% |
| Glycol Distearate | 0,4% |
| Silica | 0,2% |
| Tocopherol | 0,1% |
| Preservative | q.s. |
| Citric Acid | ad pH 5,2 |

Tab. 10: Duschöl

| | |
|---|---|
| Helianthus Annuus Seed Oil | 10,0% |
| Ricinus Communis Seed Oil | 10,0% |
| MIPA-Laureth Sulfate | 20,0% |
| Produkt Beispiel A | 15,0% |
| Laureth-4 | 0,5% |
| Cocamide DEA | 0,9% |
| Perfume (fragrance) | 0,2% |
| Prunus Amygdalus Dulcis Oil | 0,8% |
| Argania Spinosa Kernel Oil | 0,8% |
| Water | 0,5% |
| Preservative | q.s. |
| Citric Acid | ad pH 5,5 |

Tab. 11: Badeöl

| | |
|---|---|
| Water | ad 100,0% |

(fortgesetzt)

| | |
|---|---|
| Sodium Laureth Sulfate | 7,0% |
| Cocamidopropyl Betaine | 6,0% |
| Cocamide DEA | 2,5% |
| Sodium Trideceth Sulfate | 2,2% |
| Produkt Beispiel E | 2,0% |
| Perfume (fragrance) | 0,5% |
| PEG-40 Hydrogenated Castor Oil | 0,1% |
| Trideceth-9 | 0,2% |
| Sodium Lauroamphoacetate | 0,5% |
| Benzophenone-4 | 0,2% |
| Cocamide MEA | 0,4% |
| Propylene Glycol | 0,5% |
| Disodium EDTA | 0,1% |
| Sodium Chloride | 0,5% |
| Glycerin | 0,4% |
| Benzyl Alcohol | 0,4% |
| Argania Spinosa Oil | 0,2% |
| Sodium Cocoyl Glutamate | 0,3% |
| Phenoxyethanol | 0,2% |
| Xanthan Gum | 0,2% |
| Carbomer | 0,2% |
| Lactic Acid | 0,3% |
| Magnesium Chloride | 0,1% |
| Coumarin | 0,1% |
| Citric Acid | ad pH 5,2 |
| Preservative | q.s. |

Tab. 12: Creme-Dusche

| | |
|---|---|
| Water | ad 100,0% |
| Sodium Laureth Sulfate | 10,0% |
| Glycerin | 3,0% |
| Cocamidopropyl Betaine | 3,0% |
| Produkt Beispiel F | 2,0% |
| Decyl Glucoside | 1,5% |
| Perfume | q.s. |
| Glycine Soja Oil | 0,2% |
| Helianthus Annuus Seed Oil | 0,1% |
| Lecithin | 0,2% |

(fortgesetzt)

| | |
|---|---|
| Coco-Glucoside | 0,5% |
| Glyceryl Oleate | 0,3% |
| Coumarin | 0,1% |
| Preservative | q.s. |

Tab. 13: Körpershampoo

| | | |
|---|---|---|
| Phase A | Produkt Beispiel F | 3,0% |
| | Simmondsia Chinensis (Jojoba) Seed Oil | 0,7% |
| | Perfume | 0,2% |
| Phase B | Sodium Cocoamphoacetate | 4,0% |
| Phase C | Water | ad 100,0% |
| | Acrylates / C10-30 Alkyl Acrylate Crosspolymer | 0,9% |
| Phase D | Sodium Lauroyl Methyl Isethionate | 4,0% |
| | Capryl/Capramidopropyl Betaine | 2,0% |
| | Citric Acid | 1,3% |
| Phase E | Water | 10,0% |
| | Polyquaternium-7 | 0,3% |
| | Preservative | q.s. |

Tab. 14: Körpershampoo

| | | |
|---|---|---|
| Phase A | Produkt Beispiel C | 6,5% |
| | Simmondsia Chinensis (Jojoba) Seed Oil | 0,4% |
| | Perfume | 0,2% |
| Phase B | Water | ad 100,0% |
| Phase C | Sodium Cocoamphoacetate | 4,0% |
| Phase D | Water | 30,0% |
| | Acrylates/Beheneth-25 Methacrylate Copolymer | 2,0% |
| | Sodium Lauroyl Methyl Isethionate | 4,0% |
| | Disodium Lauryl Sulfosuccinate | 2,0% |
| Phase E | Preservative | q.s. |

Tab. 15: Shampoo

| | | |
|---|---|---|
| Phase A | Produkt Beispiel E | 3,0% |
| | Caprylic/Capric Triglyceride | 0,5% |
| | Perfume | 0,2% |
| Phase B | Water | ad 100,0% |
| Phase C | Sodium Cocoamphoacetate | 7,0% |

(fortgesetzt)

| | | |
|---|---|---|
| Phase D | Glycerin | 1,0% |
| | Xanthan Gum | 0,8% |
| | Water | 25,0% |
| Phase E | Water | 10,0% |
| | Acrylates/Beheneth-25 Methacrylate Copolymer | 2,0% |
| Phase F | Water | 10,0% |
| | Polyquaternium-10 | 0,2% |
| Phase G | Cocamidopropyl Betaine | 5,0% |
| | Preservative | q.s. |

Tab. 16: Shampoo

| | | |
|---|---|---|
| Phase A | Produkt Beispiel E | 8,0% |
| | Argania Spinosa Kernel Oil | 0,5% |
| Phase B | Water | ad 100,0% |
| Phase C | Perfume | 0,3% |
| | Polyglyceryl-6 Caprylate; Polyglyceryl-4 Caprate; Propylene Glycol | 2,0% |
| Phase D | Water | 20,0% |
| Phase E | Sodium Laureth Sulfate | 9,0% |
| Phase F | Water | 10,0% |
| | Cocamidopropyl Betaine | 3,0% |
| | PEG-120 Methyl Glucose Dioleate | 1,0% |
| Phase G | Water | 10,0% |
| | Sodium Chloride | 0,7% |
| | Polyquaternium-10 | 0,2% |
| Phase H | Citric Acid | ad pH 5,5 |
| Phase I | Preservative | q.s. |

Tab. 17: Duschgel

| | | |
|---|---|---|
| Phase A | Produkt Beispiel F | 3,0% |
| | Caprylic/Capric Triglyceride | 0,5% |
| | Perfume | 0,2% |
| Phase B | Water | ad 100,0% |
| Phase C | Sodium Cocoamphoacetate | 5,6% |
| Phase D | Lauryl Glucoside | 4,4% |
| Phase E | Coco-Glucoside | 1,2% |
| Phase F | Sodium/Disodium Cocoyl Glutamate | 3,6% |
| | Water | 10,0% |
| | Glycerin | 0,7% |

(fortgesetzt)

| | | |
|---|---|---|
| Phase G | Water | 10,0% |
| | Xanthan Gum | 2,0% |
| Phase H | Citric Acid | ad pH 6,0 |
| Phase I | Preservative | q.s. |

Tab. 18: Shampoo

| | | |
|---|---|---|
| Phase A | Produkt Beispiel C | 2,0% |
| | Caprylic/Capric Triglyceride | 0,5% |
| | Perfume | 0,2% |
| Phase B | Water | ad 100,0% |
| Phase C | Sodium Lauryl Sulfate | 9,0% |
| Phase D | Cocamidopropyl Betaine | 3,0% |
| Phase E | Cocamide MEA | 1,9% |
| | Xanthan Gum | 0,2% |
| | Water | 10,0% |
| Phase F | Water | 10,0% |
| | Polyquaternium-10 | 0,2% |
| Phase G | Citric Acid | ad pH 5,7 |
| Phase H | Preservative | q.s. |

Tab. 19: Deo

| | | |
|---|---|---|
| Phase A | Produkt Beispiel E | 3,0% |
| | Glycine Soja (Soybean) Oil | 0,2% |
| | Perfume | 0,1% |
| Phase B | Phenoxyethanol | 0,5% |
| | Caprylyl Glycol | 0,2% |
| Phase C | Water | 50,0% |
| | Hydroxethyl Cellulose | 0,75% |
| | Sodium Hydroxide (10% in water) | 0,25% |
| Phase D | Aluminium Chlorohydrate | 19,0% |
| Phase E | Water | ad 100,0% |

Tab. 20: Cleansing Oil Shampoo

| | |
|---|---|
| Water | ad 100,0% |
| Sodium Laureth Sulfate | 7,0% |
| MIPA-Laureth Sulfate | 4,0% |
| Sodium Chloride | 3,2% |
| Cocamidopropyl Betaine | 3,0% |

(fortgesetzt)

| | |
|---|---|
| Produkt Beispiel E | 3,0% |
| Glycerin | 2,5% |
| PEG-18 Castor Oil Dioleate | 2,0% |
| Propylene Glycol; PEG-55 Propylene Glycol Oleate | 2,0% |
| Laureth-5 Carboxylic Acid | 1,0% |
| Persea Gratissima (Avocado) Oil | 1,0% |
| Polyglyceryl-6 Caprylate; Polyglyceryl-4 Caprate; Propylene Glycol | 0,9% |
| Sodium Benzoate | 0,7% |
| Salicylic Acid | 0,3% |
| Linalool | 0,2% |
| alpha-Isomethyl lonone | 0,1% |
| Limonene | 0,1% |
| Zea Mays (Corn) Germ Oil | 0,2% |
| Argania Spinosa Oil | 0,1% |
| Camellia Oleifera Seed Oil | 0,1% |
| Sodium Hydroxide | 0,3% |
| Citric Acid | ad pH 5,0 |
| Perfume, Dyes | q.s. |

Tab. 21: Pampering Oil Bath

| | |
|---|---|
| Water | ad 100% |
| Glycine Soja Oil | 20,0% |
| Produkt Beispiel B | 10,0% |
| Polyglyceryl-3 Palmitate | 4,5% |
| Glyceryl Caprylate | 4,5% |
| Simmondsia Chinensis Seed Oil | 1,5% |
| Prunus Amygdalus Dulcis (Sweet Almond) Oil | 1,0% |
| Triticum Vulgare Germ Oil | 1,0% |
| Tocopherol | 0,2% |
| Limonene | 0,1% |
| Linalool | 0,1% |
| Citral | 0,1% |
| Dyes | q.s. |

Tab. 22: Shower Cream

| | |
|---|---|
| Water | ad 100% |
| Glycerin | 7,5% |
| Glycine Soja Oil | 3,0% |

(fortgesetzt)

| | |
|---|---|
| Lauryl Glucoside | 3,0% |
| Sodium Coco Sulfate | 3,0% |
| Produkt Beispiel C | 2,5% |
| Alcohol | 1,5% |
| Xanthan Gum | 1,5% |
| Butyrospermum Parkii Butter Extract | 1,2% |
| Sodium Cetearyl Sulfate | 1,0% |
| Sodium Cocoyl Glutamate | 1,0% |
| Disodium Cocoyl Glutamate | 1,0% |
| Tocopherol | 0,1% |
| Helianthus Annuus Seed Oil | 0,3% |
| Limonene | 0,1% |
| Benzyl Salicylate | 0,1% |
| Linalool | 0,1% |
| Dyes | q.s. |

Tab. 23: Shower Gel

| | |
|---|---|
| Water | ad 100% |
| Sodium Coco Sulfate | 5,0% |
| Glycerin | 5,0% |
| Lauryl Glucoside | 4,0% |
| Sodium Lactate | 2,5% |
| Produkt Beispiel D | 2,0% |
| Polyglyceryl-4 Caprate | 2,0% |
| Sodium Cocoyl Glutamate | 2,0% |
| Disodium Cocoyl Glutamate | 2,0% |
| Alcohol | 1,0% |
| Prunus Cerasus Fruit Extract | 1,0% |
| Polyglyceryl-6 Caprylate; Polyglyceryl-4 Caprate; Propylene Glycol | 1,0% |
| Limonene | 0,1% |
| Coumarin | 0,2% |
| Linalool | 0,1% |
| Citral | 0,1% |
| Dyes | q.s. |

Tab. 24: Liquid Soap

| | |
|---|---|
| Water | ad 100% |
| Glycerin | 7,0% |

(fortgesetzt)

| | |
|---|---|
| Alcohol | 4,0% |
| Sodium Coco Sulfate | 3,0% |
| Lauryl Glucoside | 2,5% |
| Produkt Beispiel E | 2,0% |
| Xanthan Gum | 1,5% |
| Mangifera Indica (Mango) Fruit Extract | 0,7% |
| Limonene | 0,1% |
| Linalool | 0,1% |
| Dyes | q.s. |

Tab. 25: Shampoo for Children

| | |
|---|---|
| Water | ad 100% |
| Sodium Coco Sulfate | 7,0% |
| Decyl Glucoside | 5,0% |
| Lactis Proteinum | 3,5% |
| Sorbitan Caprylate | 3,0% |
| Produkt Beispiel F | 3,0% |
| Glycerin | 2,5% |
| Sodium Lactate | 2,5% |
| Alcohol | 2,0% |
| Hydrolyzed Wheat Protein | 0,7% |
| Hydrolyzed Wheat Starch | 0,7% |
| Sodium Chloride | 0,9% |
| Limonene | 0,1% |
| Citral | 0,1% |
| Phenethyl Alcohol | 0,1% |
| Dyes | q.s. |

Tab. 26: Cream Soap

| | |
|---|---|
| Water | ad 100% |
| Alcohol | 5,0% |
| Coco-Glucoside | 5,0% |
| Glycerin | 5,0% |
| Produkt Beispiel F | 2,5% |
| Disodium Cocoyl Glutamate | 2,0% |
| Xanthan Gum | 1,5% |
| Citric Acid | ad pH 5,5 |
| Malva Sylvestris Leaf Extract | 1,0% |

(fortgesetzt)

| | |
|---|---|
| Glyceryl Oleate | 1,0% |
| Sodium Cocoyl Glutamate | 0,7% |
| Linalool | 0,1% |
| Limonene | 0,1% |
| Dyes | q.s. |

Tab. 27: Make-up Remover

| | |
|---|---|
| Sodium Cocoamphopropionate | 5,0% |
| Propylene Glycol | 35,0% |
| Produkt Beispiel F | 30,0% |
| Glycerin | 30,0% |
| Preservative | q.s. |

Tab. 28: Make-up Remover

| | |
|---|---|
| Cocamidopropyl Betaine | 8,0% |
| Water | 79,0% |
| Produkt Beispiel E | 3,0% |
| Glycerin | 10,0% |
| Citric Acid | ad pH 5,5 |
| Preservative | q.s. |

Tab. 29: Lösung für Wet Wipes

| | |
|---|---|
| Produkt Beispiel E | 1,5% |
| Perfume | 0,2% |
| Glycerin | 2,0% |
| Sodium Lactate; Sodium PCA; Glycine; Fructose; Urea; Niacinamide; Inositol; Sodium Benzoate; Lactic Acid | 0,2% |
| Water | 95,6% |
| Preservative | q.s. |

Tab. 30: Lösung für Wet Wipes

| | |
|---|---|
| Produkt Beispiel E | 2,0% |
| Isopropyl Myristate | 0,3% |
| Phenoxyethanol; Methylparaben; Ethylparaben; Butylparaben; Propylparaben; Isobutylparaben | 0,2% |
| Perfume | 0,1% |
| Propylene Glycol | 3,0% |
| Water | 94,0% |

(fortgesetzt)

| | |
|---|---|
| Cetrimonium Bromide | 0,1% |

Tab. 31: O/W Make-up remover wipe

| | | |
|---|---|---|
| Phase A | Ethylhexyl Stearate; Phenoxyethanol; Polyglyceryl-4 Laurate; Sorbitan Laurate; Dilauryl Citrate | 4,0% |
| | Cetyl Ricinoleate | 0,8% |
| Phase B | Water | ad 100,0% |
| | Glycerin | 1,5% |
| Phase C | Produkt Beispiel C | 1,0% |
| Phase D | Phenoxyethanol | 0,1% |
| | Perfume | q.s. |
| | Preservative | q.s. |

Tab. 32: Micellares Wasser

| | |
|---|---|
| Water | ad 100,0% |
| Produkt Beispiel D | 5,0% |
| Glycerin | 1,5% |
| Disodium Cocoamphodiacetate | 0,5% |
| Disodium EDTA | 0,2% |
| Polyaminopropyl Biguanide | 0,2% |
| Citric Acid, 30% | ad pH 5,5 |

Tab. 33: Micellar Solution Cleanser

| | |
|---|---|
| Water | ad 100,0% |
| Butylene Glycol | 5,0% |
| Coco-Glucoside | 2,0% |
| Produkt Beispiel D | 2,0% |
| Glycerin | 1,0% |
| Allantoin | 0,1% |
| Cistus Incanus Extract; Maltodextrin | 0,2% |
| Perfume | 0,2% |
| Citric Acid, 30% | ad pH 5,5 |

Tab. 34: Cleansing Water

| | |
|---|---|
| Water | ad 100,0% |
| Produkt Beispiel E | 2,5% |
| Phenoxyethanol; Ethylhexylglycerin | 0,9% |

(fortgesetzt)

| | |
|---|---|
| Caprylic/Capric Triglyceride | 0,5% |
| Glycerin | 0,5% |
| Disodium EDTA | 0,2% |
| Citric Acid, 30% | ad pH 5,5 |

Tab. 35: Creme Dusche

| | |
|---|---|
| Water | ad 100% |
| Ammonium Lauryl Sulfate | 10,0% |
| Produkt Beispiel D | 2,0% |
| Aloe Barbadensis Leaf Juice | 2,0% |
| Cocamidopropyl Betaine | 2,0% |
| Decyl Glucoside | 1,0% |
| Glycerin | 1,0% |
| Prunus Amygdalus Dulcis Oil | 0,5% |
| Glyceryl Oleate | 0,3% |
| Lauryl Glucoside | 0,3% |
| Coco-Glucoside | 0,4% |
| Benzyl Alcohol | 0,2% |
| Benzoic Acid | 0,3% |
| Dehydroacetic Acid | 0,2% |
| Sodium Benzoate | 0,3% |
| Potassium Sorbate | 0,2% |
| Tocopherol | 0,1% |
| Citric Acid | ad pH 4,5 |
| Perfume, Dyes | q.s. |

Tab. 36: Creme Dusche

| | |
|---|---|
| Water | ad 100% |
| Sodium Laureth Sulfate | 8,0% |
| Produkt Beispiel F | 3,0% |
| Cocamidopropyl Betaine | 3,0% |
| Glycerin | 1,0% |
| Glucose | 0,5% |
| Prunus Amygdalus Dulcis Oil | 0,7% |
| Sodium Chloride | 0,3% |
| Polyquaternium-7 | 0,3% |
| Styrene/Acrylates Copolymer | 0,4% |
| PEG-200 Hydrogenated Glyceryl Palmate; PEG-7 Glyceryl Cocoate | 0,5% |

(fortgesetzt)

| | |
|---|---|
| Citric Acid | ad pH 5,5 |
| Perfume, Dyes | q.s. |

Tab. 37: Pflegedusche

| | |
|---|---|
| Water | ad 100% |
| Sodium Laureth Sulfate | 9,0% |
| Sodium Hydroxypropyl Starch Phosphate | 2,5% |
| Produkt Beispiel E | 2,0% |
| Cocamidopropyl Betaine | 2,0% |
| Petrolatum | 1,0% |
| Sodium Cocoyl Glycinate | 1,0% |
| Lauric Acid | 0,5% |
| Sodium Lauroyl Isethionate | 0,5% |
| Glycerin | 0,4% |
| Helianthus Annuus Seed Oil | 0,3% |
| Olea Europaea Fruit Oil | 0,2% |
| Sodium Chloride | 0,4% |
| Stearic Acid | 0,5% |
| Guar Hydroxypropyltrimonium Chloride | 0,2% |
| Sodium Isethionate | 0,1% |
| Tetrasodium EDTA | 0,1% |
| Alumina | 0,1% |
| Citric Acid | ad pH 5,5 |
| Perfumes, Dyes, Preservatives | q.s. |

Tab. 38: Creme Dusche

| | |
|---|---|
| Water | ad 100% |
| Sodium Coco-Sulfate | 15,0% |
| Glycerin | 3,5% |
| Produkt Beispiel F | 3,5% |
| Glycine Soja Oil | 0,5% |
| Coco-Glucoside | 0,8% |
| Caprylic/Capric Triglyceride | 0,2% |
| Xanthan Gum | 0,8% |
| Prunus Amygdalus Dulcis Oil | 0,1% |
| Simmondsia Chinensis Seed Oil | 0,1% |
| Sodium Cocoyl Glutamate | 0,3% |
| Disodium Cocoyl Glutamate | 0,5% |

(fortgesetzt)

| | |
|---|---|
| Sodium Cetearyl Sulfate | 0,2% |
| Tocopherol | 0,1% |
| Helianthus Annuus Seed Oil | 0,1% |
| Alcohol | 0,5% |
| Citral | 0,1% |
| Geraniol | 0,1% |
| Limonene | 0,1% |
| Linalool | 0,1% |
| Citric Acid | ad pH 5,8 |
| Perfume, Dyes | q.s. |

Tab. 39: Weitere Formulierungsbeispiele

| | 39a | 39b | 39c | 39d | 39e | 39f | 39g | 39h | 39i | 39j |
|---|---|---|---|---|---|---|---|---|---|---|
| **Wasser** | ad 100% | | | | | | | | | |
| **Produkt Beispiel D** | 3,0% | 4,0% | 5,5% | 1,0% | 3,0% | 3,0% | 5,0% | 4,0% | 3,5% | 3,0% |
| **Sodium Laureth Sulfate** | 9,0% | 8,0% | 9,0% | - | - | - | - | - | - | - |
| **Sodium Lauryl Sulfate** | - | - | - | 6,0% | - | - | - | - | 3,5% | - |
| **Cocamidopropyl Betaine** | - | 2,0% | 3,0% | 7,0% | 5,0% | 6,0% | - | - | 2,0% | 7,0% |
| **Sodium Cocoamphoacetate** | 3,0% | - | - | 1,5% | 4,5% | - | 3,0% | - | 3,5% | - |
| **Lauryl Glucoside** | - | - | - | - | 3,5% | 5,0% | 3,0% | 7,0% | - | - |
| **Coco-Glucoside** | - | 2,0% | - | - | 1,5% | 1,0% | 5,5% | 2,5% | 2,0% | - |
| **Sodium Cocoyl Glutamate** | - | - | - | - | - | 1,0% | 1,7% | 5,0% | 0,5% | - |
| **Stearic Acid** | - | - | 1,0% | - | - | - | - | - | 0,1% | 3,5% |
| **Glyceryl Glucoside** | - | 0,3% | - | - | 0,3% | - | 0,2% | - | - | - |
| **Sucrose Cocoate** | 0,5% | - | 1,0% | 1,0% | 0,3% | 0,2% | - | 1,0% | 1,0% | 1,0% |
| **Glycerin** | 0,5% | 1,0% | 0,5% | - | 0,3% | 0,4% | 1,5% | 1,0% | 0,5% | 1,0% |
| **PEG-7 Glyceryl Cocoate** | - | 0,3% | - | - | - | - | - | - | - | 0,5% |
| **Trideceth-9** | - | 0,2% | - | - | 0,2% | - | - | - | - | - |
| **Polysorbate 20** | - | -0,5% | - | - | - | - | - | - | 0,3% | 0,2% |
| **PEG-40 Hydrogenated Castor Oil** | - | - | 0,3% | - | 0,5% | - | - | - | 1,0% | - |
| **PEG-6 Caprylic/Capric Glycerides** | - | - | - | - | 0,3% | - | - | - | 0,2% | 0,2% |
| **Polyglyceryl-4 Caprate** | - | - | - | 2,0% | - | 0,5% | - | 0,5% | - | 0,5% |
| **Polyquaternium-10** | - | 0,2% | - | 0,1% | - | - | - | 0,2% | 0,2% | - |
| **Hydroxypropyl Guar Hydroxypropyltrimonium Chloride** | 0,2% | - | 0,3% | 0,2% | 0,2% | 0,2% | 0,2% | 0,1 % | - | - |
| **Silicone Quaternium-22** | - | - | 0,3% | - | 0,3% | - | - | - | - | - |
| **Dimethicone** | - | 0,3% | - | - | - | - | - | - | 0,1% | - |
| **Amodimethicone** | - | 0,1% | - | 0,1% | 0,1% | - | - | - | 0,5% | - |
| **Argania Spinosa Oil** | - | - | 0,2% | 0,1% | 0,1% | - | 0,2% | - | - | - |

(fortgesetzt)

| | 39a | 39b | 39c | 39d | 39e | 39f | 39g | 39h | 39i | 39j |
|---|---|---|---|---|---|---|---|---|---|---|
| **Wasser** | ad 100% | | | | | | | | | |
| **Prunus Amygdalus Dulcis Oil** | 0,2% | - | 0,2% | 0,3% | - | 0,1% | - | - | 0,2% | 0,2% |
| **Olea Europaea Fruit Oil** | 0,2% | 0,1% | - | - | - | 0,1% | - | 0,2% | 0,1% | - |
| **Butyrospermum Parkii Butter Extract** | - | - | 0,2% | - | 0,1% | - | - | - | - | - |
| **Persea Gratissima Oil** | - | - | - | 0,1% | - | 0,1% | 0,2% | - | - | - |
| **Hydrogenated Castor Oil** | - | 0,2% | - | 0,1% | - | - | - | - | 0,1% | 0,2% |
| **Glycol Distearate** | - | 0,5% | - | - | 0,5% | - | 0,3% | - | 0,5% | 0,5% |
| **Isostearamide MIPA; Glyceryl Laurate** | 1,0% | - | - | 1,5% | - | - | 0,2% | - | 1,0% | 0,5% |
| **Cocamide DEA** | - | - | 0,5% | - | - | 1,0% | - | - | - | - |
| **Sodium Chloride** | 0,3% | 1,2% | 1,0% | 0,5% | - | 1,0% | - | 1,0% | - | 0,3% |
| **PEG-120 Methyl Glucose Dioleate** | 0,2% | 3,5% | 1,0% | - | 1,5% | - | - | - | 0,5% | - |
| **Xanthan Gum** | - | - | 0,5% | 0,8% | - | 0,7% | 2,0% | 1,0% | - | - |
| **Cellulose** | - | - | - | 0,1% | - | 0,1% | 0,1% | 0,2% | 0,1% | - |
| **Zinc Pyrithione** | - | 0,1% | - | - | - | - | - | - | 0,1% | - |
| **Benzophenone-4** | - | 0,1% | 0,1% | 0,1% | 0,1% | - | 0,1% | - | 0,1% | - |
| **Tetrasodium EDTA** | 0,1% | 0,1% | - | 0,1% | 0,1% | - | - | - | 0,1% | - |
| **Caffeine** | - | 0,1% | 0,1% | - | - | - | - | 0,1% | 0,1% | - |
| **Hydrolyzed Keratin** | - | - | 0,1% | - | - | 0,1% | 0,2% | 0,1% | 0,1% | - |
| **Panthenol** | 0,1% | 0,1% | 0,1% | 0,1% | 0,1% | 0,1% | - | 0,1% | 0,1% | 0,1% |
| **Citric Acid** | ad pH 5,5 | | | | | | | | | |
| **Perfumes, Dyes, Preservatives** | q.s. | | | | | | | | | |

Tabelle 40: Weitere Formulierungsbeispiele

| | 40a | 40b | 40c | 40d | 40e | 40f | 40g | 40h | 40i | 40j |
|---|---|---|---|---|---|---|---|---|---|---|
| **Wasser** | ad 100% | | | | | | | | | |
| **Produkt Beispiel F** | 9,0% | 5,0% | 5,0% | 4,0% | 4,0% | 2,5% | 6,0% | 4,0% | 3,0% | 3,0% |
| **Sodium Lauryl Sulfate** | - | 8,0% | 8,0% | - | - | - | - | 3,5% | - | - |
| **Coco-Betai ne** | - | 5,0% | - | 5,5% | - | - | - | 3,0% | - | - |
| **Cocamidopropyl Betaine** | - | -3,0% | 3,0% | -5,0% | 5,0% | - | - | - | 3,0% | 2,0% |
| **Sodium Cocoamphoacetate** | - | - | 2,5% | 3,0% | - | 5,0% | - | 3,0% | 4,0% | - |
| **Disodium Lauryl Sulfosuccinate** | - | - | 1,0% | - | - | - | - | 1,2% | - | - |
| **Coco-Glucoside** | - | - | - | 3,0% | 5,0% | 4,0% | 5,0% | 1,0% | - | 2,0% |
| **Sodium Cocoyl Glutamate** | - | - | - | 2,5% | - | 3,0% | 4,5% | 0,5% | 2,5% | 0,3% |
| **Stearic Acid** | - | - | 0,3% | - | - | - | - | 0,1% | - | 0,5% |
| **Sodium Cocoyl Glycinate** | - | - | - | - | 5,0% | -3,5% | 3,5% | - | 2,0% | 7,0% |
| **Sodium Lauroyl Methyl Isethionate** | - | | | 1,0% | - | 1,5% | - | 1,0% | 0,5% | 0,5% |
| **Sucrose Cocoate** | 0,5% | 0,4% | - | 1,0% | - | - | 0,2% | 0,3% | 1,0% | 0,3% |
| **Glycerin** | 1,5% | 0,3% | 0,5% | 0,5% | 0,3% | 0,5% | 1,0% | 0,5% | 0,3% | 1,0% |
| **PEG-40 Hydrogenated Castor Oil** | - | 1,0% | - | - | - | - | - | 0,3% | - | - |
| **Polyglyceryl-4 Caprate** | 0,5% | - | - | 0,5% | - | 2,6% | - | - | 1,1% | - |
| **Polyquaternium-11** | - | 0,2% | - | - | 0,1% | - | - | 0,2% | - | 0,3% |
| **Guar Hydroxypropyltrimonium Chloride** | - | - | 0,3% | 0,2% | 0,2% | 0,3% | 0,2% | 0,1% | 0,2% | - |
| **Dimethicone** | - | 0,3% | - | - | - | - | - | 0,2% | - | - |
| **Aminopropyl Dimethicone** | - | 0,3% | 0,5% | - | - | - | - | 0,3% | - | - |
| **Helianthus Annuus Seed Oil** | 0,3% | - | 0,1% | 0,5% | - | 0,1% | 0,1% | 0,2% | - | 0,1% |
| **Olea Europaea Fruit Oil** | 0,2% | 0,1% | 0,2% | - | 0,2% | 0,1% | 0,2% | - | 0,6% | 0,2% |
| **PEG-3 Distearate** | - | 0,5% | - | - | - | - | - | 0,5% | - | - |
| **Acrylates / C10-30 Alkyl Acrylate Crosspolymer** | 0,5% | - | 0,4% | - | 0,5% | 0,4% | - | - | 0,5% | - |
| **Sodium Hydroxide, 25%** | 0,6% | - | 0,6% | - | 0,8% | 0,5% | - | - | 0,7% | - |

(fortgesetzt)

| | 40a | 40b | 40c | 40d | 40e | 40f | 40g | 40h | 40i | 40j |
|---|---|---|---|---|---|---|---|---|---|---|
| **Wasser** | ad 100% | | | | | | | | | |
| **Cocamide MEA** | - | 0,8% | 1,0% | 1,0% | - | 0,2% | 0,6% | 1,0% | - | 0,3% |
| **Sodium Chloride** | 0,2% | 0,7% | 0,2% | - | 0,2% | 0,1% | - | 1,0% | 0,2% | 1,0% |
| **Propylene Glycol; PEG-55 Propylene Glycol Oleate** | - | 2,5% | - | - | - | - | - | 0,8% | - | - |
| **Xanthan Gum** | 0,2% | - | 0,2% | 1,5% | 0,5% | - | 1,8% | 0,2% | 1,1% | 0,9% |
| **Hydroxyethyl Ethylcellulose** | 0,1% | - | - | 0,1% | - | 0,1% | 0,1% | - | - | - |
| **Benzophenone-4** | - | 0,1% | 0,2% | - | - | - | - | 0,2% | - | 0,1% |
| **Menthol** | 0,1% | - | 0,1% | - | - | 0,1% | - | 0,1% | 0,1% | 0,1% |
| **Caffeine** | - | - | 0,1% | - | 0,1% | - | - | 0,1% | - | 0,1% |
| **Benzyl Alcohol** | 0,1% | - | - | - | - | 0,1% | - | 0,1% | - | - |
| **Coumarin** | 0,1% | - | 0,1% | 0,1% | - | - | 0,1% | 0,1% | - | 0,1% |
| **Hydrolyzed Wheat Protein** | - | - | 0,1% | 0,1% | 0,1% | 0,2% | 0,2% | 0,1% | - | - |
| **Panthenol** | 0,1% | 0,1% | - | 0,1% | 0,1% | 0,1% | 0,1% | - | 0,1% | 0,1% |
| **Citric Acid** | ad pH 5,2 | | | | | | | | | |
| **Perfumes, Dyes, Preservatives** | q.s. | | | | | | | | | |

Tabelle 41: Weitere Formulierungsbeispiele

| | 41a | 41b | 41c | 41d | 41e | 41f | 41g | 41h | 41i | 41j |
|---|---|---|---|---|---|---|---|---|---|---|
| **Wasser** | ad 100% | | | | | | | | | |
| **Produkt Beispiel E** | 3,5% | 3,0% | 2,5% | 4,0% | 3,0% | 4,0% | 6,0% | 4,0% | 5,0% | 4,0% |
| **MIPA-Laureth Sulfate** | 10,5% | 5,0% | - | - | - | - | - | - | - | - |
| **Sodium C14-16 Olefin Sulfonate** | - | - | 8,0% | - | - | - | - | - | 3,5% | - |
| **Coco-Betai ne** | - | 4,0% | - | 5,5% | - | - | 4,0% | | 3,5% | - |
| **Cocamidopropyl Betaine** | 2,0% | - | 4,0% | - | 5,0% | 5,0% | - | - | - | 4,0% |
| **Sodium Cocoamphopropionate** | - | - | 1,0% | - | 2,0% | - | 1,5% | 4,0% | 2,0% | 3,5% |
| **Coco-Glucoside** | - | 2,5% | - | 2,5% | 3,0% | - | 4,5% | 3,5% | 2,0% | 0,5% |
| **Sodium Cocoyl Glutamate** | - | - | - | 1,5% | 1,0% | 1,5% | 1,5% | 1,5% | 0,8% | 0,3% |
| **Lauric Acid** | - | 0,5% | - | - | - | 1,0% | - | 2,0% | - | 4,5% |
| **Sodium Cocoyl Glycinate** | - | - | - | 2,5% | - | 5,0% | - | - | 0,8% | 0,5% |
| **Sodium Cocoyl Sarcosinate** | - | 0,7% | 0,5% | - | - | 1,0% | 0,5% | - | - | - |
| **Dicaprylylether** | 0,5% | - | - | - | - | 0,2% | - | - | - | - |
| **Glycerin** | 0,5% | 0,3% | 0,5% | 1,5% | 0,4% | 0,5% | 1,0% | 0,5% | 0,3% | 1,0% |
| **Polysorbate 20** | 0,5% | - | - | - | - | 0,5% | - | - | - | 0,4% |
| **Polyglyceryl-4 Laurate** | 0,5% | - | 0,4% | 0,3% | - | - | 0,5% | - | 1,1% | - |
| **Polyquaternium-37** | 0,4% | - | - | 0,1% | - | - | - | 0,2% | 0,1% | - |
| **Hydroxypropyl Guar Hydroxypropyltrimonium Chloride** | - | 0,2% | 0,1% | 0,2% | - | - | 0,3% | - | 0,2% | - |
| **Cassia Hydroxypropyltrimonium Chloride** | - | 0,1% | - | - | 0,2% | - | - | - | - | - |
| **Sodium Hydroxypropyl Starch Phosphate** | 0,2% | - | - | - | - | 0,4% | - | - | - | 0,5% |
| **Hydroxypropyl Methylcellulose** | 0,2% | - | - | 0,2% | 0,2% | - | - | - | 0,2% | - |
| **Dimethicone** | 0,2% | - | - | - | - | 1,0% | - | - | 0,3% | - |
| **Aminopropyl Dimethicone** | 0,2% | 0,4% | - | - | - | 0,5% | - | - | - | 0,5% |
| **Palmitamidopropyltrimonium Chloride** | 0,3% | - | 0,5% | - | - | - | - | - | 0,4% | - |
| **Persea Gratissima (Avocado) Oil** | 0,1% | 1,1% | - | 0,1% | 0,2% | - | 0,3% | - | 0,1% | 0,1% |

(fortgesetzt)

| | 41a | 41b | 41c | 41d | 41e | 41f | 41g | 41h | 41i | 41j |
|---|---|---|---|---|---|---|---|---|---|---|
| **Wasser** | ad 100% | | | | | | | | | |
| **Butyrospermum Parkii Butter Extract** | 0,2% | - | 0,1% | - | - | 0,3% | - | - | - | - |
| **Prunus Amygdalus Dulcis Oil** | 0,2% | - | 0,2% | 0,2% | - | - | 0,2% | 0,5% | - | - |
| **Glycol Distearate** | 0,5% | 0,7% | - | - | 0,5% | 0,8% | - | - | 0,4% | 0,3% |
| **Carbomer** | - | 0,3% | - | - | - | 0,5% | 0,7% | - | 0,5% | - |
| **Sodium Hydroxide, 25%** | - | 0,5% | - | - | - | 0,7% | 1,0% | - | 0,8% | - |
| **Isostearamide MIPA; Glyceryl Laurate** | 0,7% | - | 0,8% | - | 0,3% | 0,3% | - | 0,4% | - | 0,3% |
| **Sorbitan Sesquicaprylate** | - | - | 0,2% | 1,0% | - | - | 0,4% | 1,0% | - | 0,7% |
| **Sodium Chloride** | 0,8% | - | 0,3% | 0,2% | 0,5% | - | - | 2,0% | - | - |
| **PEG-18 Glyceryl Oleate/Cocoate** | 0,8% | - | - | - | - | 0,6% | - | - | - | 0,9% |
| **Xanthan Gum** | - | 1,0% | 0,2% | 1,0% | - | 0,2% | 0,3% | - | - | 0,2% |
| **Algin** | - | 0,2% | - | - | 1,0% | - | - | - | 1,2% | - |
| **Caragenaan** | 0,5% | - | - | 0,3% | - | - | - | 0,3% | - | 0,2% |
| **Silica** | 0,1% | - | 0,1% | - | - | 0,2% | - | - | 0,2% | 0,1% |
| **Cetearyl Alcohol** | 0,2% | - | - | - | 0,3% | 0,2% | - | - | - | 0,3% |
| **Benzophenone-4** | 0,1% | 0,1% | 0,2% | - | 0,2% | 0,2% | - | - | - | 0,1% |
| **Tetrasodium EDTA** | 0,1% | 0,1% | - | 0,1% | 0,1% | 0,2% | 0,1% | - | 0,2% | 0,1% |
| **Octopirox** | 0,2% | - | - | - | - | 0,1% | - | - | - | - |
| **Zinc PCA** | - | - | 0,1% | - | - | - | - | - | - | 0,1% |
| **Creatine** | 0,1% | - | 0,1% | 0,1% | - | 0,1% | 0,1% | 0,1% | - | 0,1% |
| **Hydrolyzed Collagen** | - | - | - | 0,1% | - | 0,1% | - | 0,1% | 0,1% | 0,1% |
| **Salicylic Acid** | 0,1% | - | - | - | 0,1% | 0,1% | - | - | 0,1% | 0,1% |
| **Panthenol** | 0,1% | 0,1% | - | 0,1% | 0,1% | 0,1% | 0,1% | - | 0,1% | 0,1% |
| **Lactic Acid** | 0,2% | - | 0,2% | - | 0,1% | 0,5% | 0,3% | - | 0,2% | - |
| **PEG-14M** | 0,3% | - | - | - | - | 0,4% | - | - | - | 0,3% |

(fortgesetzt)

| | 41a | 41b | 41c | 41d | 41e | 41f | 41g | 41h | 41i | 41j |
|---|---|---|---|---|---|---|---|---|---|---|
| **Wasser** | ad 100% | | | | | | | | | |
| **1,2-Hexanediol** | 0,3% | - | - | - | - | 0,3% | - | 0,2% | - | - |
| **Citric Acid** | ad pH 5,5 | | | | | | | | | |
| **Perfumes, Dyes, Preservatives** | q.s. | | | | | | | | | |

**Tabelle 42: Liste der eingesetzten Rohstoffe**

| INCI | Handelsname, Firma |
|---|---|
| 1,2-Hexanediol | Hydrolite-6 841129, Symrise |
| Acrylates/Beheneth-25 Methacrylate Copolymer | Novethix L-10 Polymer, Lubrizol |
| Acrylates / C10-30 Alkyl Acrylate Crosspolymer | TEGO Carbomer 841 SER, Evonik Industries AG, 100% |
| Algin | Hydagen 558 P, BASF |
| Allantoin | Allantoin, DSM Nutritional Products, Inc. |
| Aloe Barbadensis Leaf Juice | Aloe-Con UP 40, Florida Food Products Inc. |
| alpha-Isomethyl lonone | alpha-Isomethylionone, Chemos GmbH |
| Alumina | Aeroxide Alu C, Evonik Industries AG |
| Aluminum Chlorohydrate | Locron L, Clariant |
| Ammonium Lauryl Sulfate | Empicol AL 70, Albright & Wilson UK Limited |
| Aminopropyl Dimethicone | ABIL Soft AF 200, Evonik Industries AG |
| Amodimethicone | DC 949, Dow Corning, 100% |
| Argania Spinosa Oil (Argania Spinosa Kernel Oil) | Argan Oil, DSM Nutritional Products Ltd. |
| Benzophenone-4 | Uvinul MS 40, BASF Corporation |
| Benzoic Acid | OriStar BZA, Orient Stars LLC |
| Benzyl Alcohol | Microcare BNA, THOR PERSONAL CARE SAS |
| Benzyl Salicylate | Seridefrizz Intense, Cheemyunion Quimica Ltda. |
| Butylene Glycol | Butylene Glycol, Oxea Corparation |
| Butyrospermum Parkii Butter Extract | Cosmosil 600, International Cosmetic Science Centre |
| Caffeine | Caffeine, Merck KGaA/EMD Chemicals, Inc. |
| Camellia Oleifera Seed Oil | Camellia Sasanqua Oil, Ikeda Corporation |
| Caprylyl Glycol | Sensiva SC 10, Schülke& Mayr GmbH |
| Capryl/Capramidopropyl Betaine | TEGO Betaine 810, Evonik Industries AG, 38% |
| Caprylic/Capric Triglyceride | TEGOSOFT CT, Evonik Industries AG, 100% |
| Carrageenan | Genugel Carrageenan, CP Kelco |
| Carbomer | TEGO Carbomer 140, Evonik Industries AG, 100% |
| Cassia Hydroxypropyltrimonium Chloride | Formularbeginn Sensomer ST 250-Polymer, Lubrizol Formularende |
| Cellulose | Arbocel A300, J. Rettenmaier & Söhne |
| Cetearyl Alcohol | TEGO Alkanol 1618, Evonik Industries |
| | AG, 100% |
| Cetyl Ricinoleate | TEGOSOFT CR, Evonik Industries AG, 100% |
| Cetrimonium Bromide | Rhodaquat M-242B/99, Rhodia |
| Chamomilla Recutita (Matricaria) Extract | Recentia CR, AkzoNobel Global Personal Care |
| Citral | Citral FF, Symrise AG |

(fortgesetzt)

| INCI | Handelsname, Firma |
|---|---|
| Citric Acid | Citric Acid USP Granular, DSM Nutritional Products, Inc. |
| Citrus Aurantifolia (Lime) Oil | AEC Lime Oil, A & E Connock, Perfumery & Cosmetics Ltd. |
| Cistus Incanus Extract; Maltodextrin | TEGO Cistus, Evonik Industries AG |
| Cocamide DEA | REWOMID DC 212 S, Evonik Industries AG, 100% |
| Cocamide MEA | REWOMID D 212, Evonik Industries AG, 100% |
| Cocamidopropyl Betaine | TEGO Betain F 50, Evonik Industries AG, 38% |
| Coco-Glucoside | Plantacare 818 UP, BASF Cognis, 51% |
| Coco-Betaine | Dehyton AB 30, BASF Cognis, 31% |
| Coumarin | Rhodiascent extra pure, Rhodia Organics |
| Creatine | TEGO Cosmo C 100, Evonik Industries AG, 100% |
| Decyl Glucoside | Plantacare 2000 UP, BASF Cognis |
| Dicaprylylether | Cetiol OE, BASF Cognis |
| Dehydroacetic Acid | Unisept DHA (Universal Preserv-A-Chem, Inc.) |
| Dimethicone | DC 200 Fluid 100 cSt, Dow Corning, 100% |
| Disodium Cocoamphodiacetate | Rewoteric AM 2 C NM, Evonik Industries AG |
| Disodium Cocoyl Glutamate | Planatpon ACG LC, BASF Cognis |
| Disodium EDTA | Dissolvine NA-2-P, AkzoNobel Global Personal Care |
| Disodium Lauryl Sulfosuccinate | REWOPOL SB F 12 P, Evonik Industries AG, 95% |
| Ethylhexyl Stearate; Phenoxyethanol; Polyglyceryl-4 Laurate; Sorbitan Laurate; Dilauryl Citrate | TEGO Wipe Flex, Evonik Industries AG |
| Geraniol | Nerol 800, International Flavors & Fragrances Inc. |
| Glucose | Organic Biovert Substrate, Lonza |
| Glycerin | Glycerol EP, vegetable, Spiga Nord, 99,7% |
| Glyceryl Caprylate | Dermosoft GMCY, Dr. Straetmans |
| Glyceryl Glucoside | Hydagen GG, BASF Cognis |
| Glyceryl Oleate | TEGIN O V, Evonik Industries AG |
| Glycine Soja (Soybean) Oil | Cropure Soybean, Croda Europe, Ltd. |
| Glycol Distearate | TEGIN G 1100, Evonik Industries AG, 100% |
| Guar Hydroxypropyltrimonium Chloride | Cosmedia Guar C 261, BASF Personal Care and Nutrition Gmbh / Jaguar C-17, Rhodia Inc. und andere |
| Helianthus Annuus (Sunflower) Seed Oil | AEC Sunflower Oil, A & E Connock, Perfumery & Cosmetics Ltd. |

(fortgesetzt)

| INCI | Handelsname, Firma |
|---|---|
| Hydrogenated Castor Oil | Cutina HR Powder, BASF Personal Care and Nutrition Gmbh |
| Hydrolyzed Collagen | Nutrilan H, BASF Personal Care and Nutrition Gmbh |
| Hydrolyzed Keratin | Kerasol, Croda, Inc. |
| Hydrolyzed Wheat Protein | Gluadin WLM, BASF Cognis |
| Hydrolyzed Wheat Starch | Cropeptide W, Croda, Inc. |
| Hydroxyethyl Ethylcellulose | Structure Cel 4400 E, AkzoNobel Global Personal Care |
| Hydroxypropyl Guar Hydroxypropyltrimonium Chloride | Jaguar C-162, Rhodia, 100% |
| Hydroxypropyl Methylcellulose | TEGOCEL HPM 50, Evonik Industries AG, 100% |
| Isopropyl Myristate | TEGOSOFT M, Evonik Industries AG, 100% |
| Isostearamide MIPA; Glyceryl Laurate | ANTIL SPA 80, Evonik Industries AG, 100% |
| Lactic Acid | AEC Lactic Acid, A & E Connock, Perfumery & Cosmetics Ltd. |
| Lactis Proteinum | AEC Whey Protein, A & E Connock, Perfumery & Cosmetics Ltd. |
| Laureth-4 | TEGO Alkanol L 4, Evonik Industries AG, 100% |
| Laureth-5 Carboxylic Acid | Marlowet 1072, Sasol Germany GmbH - Marl |
| Lauric Acid | Prifrac 2920, Croda Europe, Ltd. |
| Lauryl Glucoside | Plantacare 1200 UP, BASF Cognis, 50% |
| Lecithin | AEC Lecithin Powder, A & E Connock, Perfumery & Cosmetics Ltd. |
| Limonene | Dipentene No. 122, Hercules Inc. |
| Linalool | Lipofresh, Lipo Chemicals, Inc. |
| Magnesium Chloride | OriStar MCL, Orient Stars LLC |
| Maltodextrin | Farmal MD 10, Corn Products International |
| Malva Sylvestris (Mallow) Leaf Extract | Herbasec Mallow Leaves, Cosmetochem International AG |
| Mangifera Indica (Mango) Fruit Extract | Mango Extract, Draco Natural Products |
| Menthol | OriStar MC, Orient Stars LLC |
| MIPA-Laureth Sulfate | Zetesol 2056, Zschimmer & Schwarz GmbH |
| Octopirox | Octopirox, Clariant Intl. Ltd. |
| Olea Europaea (Olive) Fruit Oil | Cropure Olive, Croda Europe, Ltd. |
| Palmitamidopropyltrimonium Chloride | VARISOFT PATC, Evonik Industries AG, 60% |
| Panthenol | D-Panthenol USP, BASF, 100% |
| PEG-120 Methyl Glucose Dioleate | ANTIL 120 Plus, Evonik Industries AG, 100% |
| PEG-14M | Polyox WSR-205, The Dow Chemical Company |
| PEG-18 Castor Oil Dioleate | Marlowet CG, Sasol Germany GmbH |

(fortgesetzt)

| INCI | Handelsname, Firma |
|---|---|
| PEG-18 Glyceryl Oleate/Cocoate | ANTIL 171, Evonik Industries AG, 100% |
| PEG-200 Hydrogenated Glyceryl Palmate; PEG-7 Glyceryl Cocoate | REWODERM LI S 80, Evonik Industries AG, 100% |
| PEG-3 Distearate | TEGIN D 1102, Evonik Industries AG, 100%; Cutina TS, BASF Cognis, 100% |
| PEG-40 Hydrogenated Castor Oil | TAGAT CH 40, Evonik Industries AG, 100% |
| PEG-40 Sorbitan Peroleate | Arlatone T, Croda |
| PEG-6 Caprylic/Capric Glycerides | TEGOSOFT GMC-6, Evonik Industries AG, 100% |
| PEG-7 Glyceryl Cocoate | TEGOSOFT GC, Evonik Industries AG, 100% |
| Persea Gratissima (Avocado) Oil | Cropure Avocado, Croda Europe, Ltd. |
| Petrolatum | Merkur 115, Sasol Wax GmbH |
| Phenethyl Alcohol | Etaphen, Vevy Europe SpA |
| Phenoxyethanol | S&M Phenoxyethanol, Schülke & Mayr GmbH |
| Phenoxyethanol; Ethylhexyl Glycerin | Euxyl PE 9010, Schülke & Mayr GmbH |
| Phenoxyethanol; Methylparaben; Ethylparaben; Butylparaben; Propylparaben; Isobutylparaben | Euxyl K 300, Schuelke & Mayr GmbH |
| Polyaminopropyl Biguanide | Microcare MBG, Thor |
| Polyglyceryl-3 Palmitate | Dermofeel PP, Dr. Straetmans |
| Polyglyceryl-4 Caprate | TEGOSOFT PC 41, Evonik Industries AG, 100% |
| Polyglyceryl-6 Caprylate; Polyglyceryl-4 Caprate; Propylene Glycol | TEGO Solve 55, Evonik Industries AG, |
| Polyquaternium-10 | Polymer JR 400, Amerchol, 100% |
| Polyquaternium-11 | Dehyquart CC 11, BASF Personal Care and Nutrition Gmbh / Luviquat PQ 11 PN, BASF Corporation |
| Polyquaternium-37 | Cosmedia Ultragel 300, BASF Personal Care and Nutrition Gmbh |
| Polyquaternium-7 | Merquat 550, Nalco, 100% |
| Polysorbate 20 | TEGO SML 20, Evonik Industries AG, 100% |
| Potassium Sorbate | Euxyl K 712, Schülke & Mayr GmbH |
| Propylene Glycol | Euxyl K 320, Schülke & Mayr GmbH |
| Propylene Glycol; PEG-55 Propylene Glycol Oleate | ANTIL 141 Liquid, Evonik Industries AG |
| Prunus Amygdalus Dulcis (Sweet Almond) Oil | Cropure Almond, Croda Europe, Ltd. |
| Prunus Cerasus (Bitter Cherry) Fruit Extract | Prunus Cerasus Fruit, Kirschen Extract, Botanica GmbH |
| Ricinus Communis Seed Oil | Lipovol CO, Lipo Chemicals |
| Salicylic Acid | OriStar SCA, Orient Stars LLC |
| Silica | Aerosil 130, Evonik Degussa GmbH |
| Silicone Quaternium-22 | ABIL T Quat 60, Evonik Industries AG, 65% |

(fortgesetzt)

| INCI | Handelsname, Firma |
|---|---|
| Silicone Quaternium-22; Polyglycerin-3 Caprate; Dipropylene Glycol; Cocamidopropyl Betaine | ABIL ME 45, Evonik Industries AG, 30% |
| Simmondsia Chinensis (Jojoba) Seed Oil | AEC Jojoba Oil Refined, A & E Connock, Perfumery & Cosmetics Ltd. |
| Sodium Benzoate | Euxyl K 712, Schülke & Mayr GmbH |
| Sodium C14-16 Olefin Sulfonate | Bioterge AS-40 AOS, Stepan, |
| Sodium Cetearyl Sulfate | Lanette E, BASF Personal Care and Nutrition Gmbh |
| Sodium Cocoamphoacetate | REWOTERIC AM C, Evonik Industries AG, 32% |
| Sodium Cocoamphopropionate | REWOTERIC AM KSF 40, Evonik Industries AG, 40% |
| Sodium Coco-Sulfate | Texapon HC G, BASF |
| Sodium Cocoyl Glutamate | Plantapon ACG HC, BASF Cognis |
| Sodium Cocoyl Glycinate | Hostapon SG, Clariant; Amilite GCS-11, Ajinomoto |
| Sodium Cocoyl Sarcosinate | Crodasinic CS, Croda |
| Sodium/Disodium Cocoyl Glutamate | PERLASTAN SC 25 NKW, Schill&Seilacher, 25%-ig, |
| Sodium Hydroxide | Unichem SOHYD, Universal Preserv-A-Chem, Inc. |
| Sodium Hydroxypropyl Starch Phosphate | Pure-Gel, Grain Processing Corporation |
| Sodium Isethionate | Hostapon SI, Company Clariant International Ltd, |
| Sodium Lactate | Sodium Lactate Solution About 50%, Merck KGaA/EMD Chemicals, Inc. |
| Sodium Lactate; Sodium PCA; Glycine; Fructose; Urea; Niacinamide; Inositol; Sodium Benzoate; Lactic Acid | LACTIL, Evonik Industries AG |
| Sodium Laureth Sulfate | Texapon NSO, BASF Cognis, 28% |
| Sodium Lauroamphoacetate | ColaTeric SLAA, Colonial Chemical Inc |
| Sodium Lauroyl Isethionate | Yongan SLI , Huanggang Yongan Pharmaceutical Co.,Ltd |
| Sodium Lauroyl Methyl Isethionate | Iselux, Innospec Active Chemicals |
| Sodium Lauryl Sulfate | Texapon LS 35, BASF Cognis, 30% |
| Sodium Trideceth Sulfate | Rhodapex EST-30, Rhodia |
| Sorbitan Caprylate | Sorbon S-10, Toho Chemical Industry Co., Ltd. |
| Sorbitan Sesquicaprylate | ANTIL Soft SC, Evonik Industries AG, 100% |
| Stearic Acid | Pristerene 4922, Croda Europe, Ltd. |
| Styrene/Acrylates Copolymer | Acudyne HS, The Dow Chemical Company |
| Sucrose Cocoate | TEGOSOFT LSE 65 K, Evonik Industries AG, 100% |
| Tetrasodium EDTA | Versene 100, The Dow Chemical Company |

(fortgesetzt)

| INCI | Handelsname, Firma |
|---|---|
| Tocopherol | Euxyl K 700, Schülke & Mayr GmbH |
| Trideceth-9 | Marlipal O 13/90, Sasol Germany GmbH - Marl |
| Triticum Vulgare Germ Oil | Cropure Wheatgerm, Croda Europe, Ltd. |
| Xanthan Gum | Keltrol CG-SFT, CP Kelco, 100% |
| Zea Mays (Corn) Germ Oil | AEC Corn Germ Oil, A & E Connock, Perfumery & Cosmetics Ltd. |
| Zinc PCA | Zincidone, UCIB, Solabia Group |
| Zinc Pyrithione | Microcare ZP, THOR PERSONAL CARE SAS |

**Patentansprüche**

1. Polyglycerinpartialester der allgemeinen Formel I

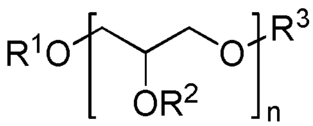

allgemeine Formel I

mit

n = 2 bis 16, bevorzugt 4 - 14, besonders bevorzugt 5-11
$R^1$, $R^2$, $R^3$ = unabhängig voneinander gleich oder verschieden ausgewählt aus H, $R^4$ und $R^5$, wobei
$R^4$ = keine Hydroxylgruppe enthaltender, gesättigter oder ungesättigter Acylrest mit 6 - 22 C-Atomen, bevorzugt mit 8 - 18 C-Atomen,
$R^5$ = mindestens eine Hydroxylgruppe enthaltender, gesättigter oder ungesättigter Acylrest mit 6 - 22 C-Atomen, bevorzugt mit 14 - 22 C-Atomen oder ein Acylrest eines Oligomers von mindestens eine Hydroxylgruppe enthaltenden, gesättigten oder ungesättigten Acylresten mit 6 - 22 C-Atomen, bevorzugt mit 14 - 22 C-Atomen,
**dadurch gekennzeichnet, dass** das Molverhältnis der Acylreste $R^4$ zu $R^5$ in einem Bereich von 95:5 bis 50:50 liegt.

2. Polyglycerinpartialester gemäß Anspruch 1, **dadurch gekennzeichnet, dass** $R^4$ und $R^5$ Acylreste von Fettsäuren sind.

3. Polyglycerinpartialester gemäß Anspruch 1 oder 2, **dadurch gekennzeichnet, dass** bezogen auf alle Reste $R^4$ im Polyglycerinpartialester mindestens 50 Mol-% der Acylreste $R^4$ ausgewählt sind aus Capryloyl-, Caproyl- und Lauroylresten.

4. Polyglycerinpartialester gemäß mindestens einem der vorherigen Ansprüche, **dadurch gekennzeichnet, dass** $R^5$ ausgewählt ist aus Ricinoyl- und Hydroxystearoylresten, ihren Oligomeren und Mischungen davon.

5. Polyglycerinpartialester gemäß mindestens einem der vorherigen Ansprüche, **dadurch gekennzeichnet, dass** bezogen auf alle Reste $R^5$ im Polyglycerinpartialester mindestens 90 Mol-% der Acylreste $R^5$ aus Ricinoylresten oder einer Mischung von Ricinoyl- und Hydroxystearoylresten bestehen.

6. Polyglycerinpartialester mindestens einem der Ansprüche 1 bis 4, **dadurch gekennzeichnet, dass** $R^5$ ausgewählt ist aus Ricinoylresten.

7. Polyglycerinpartialester gemäß mindestens einem der vorherigen Ansprüche, **dadurch gekennzeichnet, dass** das Gewichtsverhältnis des Polyglycerylrests zur Summe der Acylreste $R^4$ und $R^5$ 85:15 bis 55:45 beträgt.

**8.** Verfahren zur Herstellung von Polyglycerinpartialestern gemäß Anspruch 1 umfassend die Verfahrensschritte:

A) Bereitstellen eines Polyglycerins mit einem mittleren Polymerisationsgrad n = 2 bis 16,
B) Acylierung eines Teils der Hydroxygruppen des Polyglycerins mit
mindestens einem ersten Carbonsäurederivat einer oder mehrerer ersten, gesättigten oder ungesättigten, keine Hydroxygruppe enthaltenden Carbonsäuren mit 6 - 22 C-Atomen und
mindestens einem zweiten Carbonsäurederivat, einer oder mehrerer zweiten, gesättigten oder ungesättigten, mindestens eine Hydroxygruppe enthaltenden Carbonsäuren mit 6 - 22 C-Atomen oder eines Oligomers der zweiten Carbonsäure,
wobei die Carbonsäurederivate ausgewählt sind aus Carbonsäuren und Carbonsäureestern,

wobei das molare Verhältnis der Acylreste des in Verfahrensschritt B) eingesetzten ersten Carbonsäurederivats zu denen des zweiten Carbonsäurederivats in einem Bereich von 95:5 bis 50:50 liegt.

**9.** Verfahren gemäß Anspruch 8 oder 9, **dadurch gekennzeichnet, dass** in Verfahrensschritt B)
mindestens eine erste Carbonsäure und mindestens eine zweite Carbonsäure, mindestens ein erster Carbonsäureester und mindestens eine zweite Carbonsäure,
mindestens eine erste Carbonsäure und mindestens ein zweiter Carbonsäureester,
mindestens ein erster Carbonsäureester und mindestens ein zweiter Carbonsäureester,
mindestens eine erste Carbonsäure und mindestens ein erster Carbonsäureester und mindestens eine zweite Carbonsäure,
mindestens eine erste Carbonsäure und mindestens ein erster Carbonsäureester und mindestens ein zweiter Carbonsäureester,
mindestens eine erste Carbonsäure und mindestens eine zweite Carbonsäure und mindestens ein zweiter Carbonsäureester,
mindestens ein erster Carbonsäureester und mindestens eine zweite Carbonsäure und mindestens ein zweiter Carbonsäureester
oder
mindestens eine erste Carbonsäure und mindestens ein erster Carbonsäureester und mindestens eine zweite Carbonsäure und mindestens ein zweiter Carbonsäureester,
eingesetzt werden.

**10.** Verfahren gemäß Anspruch 8 oder 9, **dadurch gekennzeichnet, dass** bezogen auf die Acylreste aller ersten Carbonsäurederivate mindestens 50 Mol-% der ersten Carbonsäuren ausgewählt sind aus Caprylsäure, Caprinsäure und Laurinsäure.

**11.** Verfahren gemäß mindestens einem der Ansprüche 8 bis 10, **dadurch gekennzeichnet, dass** bezogen auf die Acylreste aller zweiten Carbonsäurederivate mindestens 90 Mol-% der zweiten Carbonsäure ausgewählt sind aus Rizinolsäure und Hydroxystearinsäure.

**12.** Verfahren gemäß mindestens einem der Ansprüche 8 bis 11, **dadurch gekennzeichnet, dass** bezogen auf die Acylreste aller zweiten Carbonsäurederivate mindestens 90 Mol-% der zweiten Carbonsäuren aus Rizinolsäure und Hydroxystearinsäure bestehen, und die zweiten Carbonsäuren ein Molverhältnis von Rizinolsäureresten zu Hydroxystearinsäureresten in einem Bereich von 100 zu 0,1 bis 50 zu 50 aufweist.

**13.** Verfahren gemäß mindestens einem der Ansprüche 8 bis 12, **dadurch gekennzeichnet, dass** das Gewichtsverhältnis des Polyglycerins zur Summe der Acylreste der eingesetzten ersten und zweiten Carbonsäurederivate 85:15 bis 55:45 beträgt.

**14.** Verfahren gemäß mindestens einem der Ansprüche 8 bis 13, **dadurch gekennzeichnet, dass** das Molverhältnis der Acylreste von gesättigten zu ungesättigten Carbonsäurederivaten, die in Verfahrensschritt B) eingesetzt werden, 99:1 - 1:99 beträgt.

**15.** Formulierung enthaltend mindestens einen Polyglycerinpartialester gemäß mindestens einem der Ansprüche 1 bis 7.

**16.** Formulierung gemäß Anspruch 15 enthaltend 0,1 Gew.-% bis 40 Gew.-% Polyglycerinpartialester gemäß mindestens einem der Ansprüche 1 bis 7 0,01 Gew.-% bis 40 Gew.-% öllösliche Substanz und 10 Gew.-% bis 98 Gew.-% Wasser.

**17.** Verwendung mindestens eines Polyglycerinpartialesters gemäß mindestens einem der Ansprüche 1 bis 7 zur Solubilisierung von mindestens einer öllöslichen Substanz in Wasser.

## Claims

**1.** Polyglycerol partial ester of the general formula I

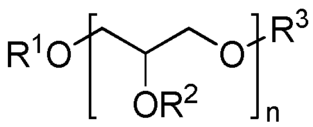

general formula I

where

n = 2 to 16, preferably 4 to 14, particularly preferably 5 to 11,
$R^1$, $R^2$, $R^3$ = independently of one another, identical or different, selected from H, $R^4$ and $R^5$, where
$R^4$ =saturated or unsaturated acyl residue having 6 - 22 carbon atoms, preferably 8 - 18 carbon atoms, comprising no hydroxyl groups,
$R^5$ =saturated or unsaturated acyl residue having 6 - 22 carbon atoms, preferably 14 - 22 carbon atoms, comprising at least one hydroxyl group or an acyl residue of an oligomer of saturated or unsaturated acyl residues having 6 - 22 carbon atoms, preferably 14 - 22 carbon atoms, comprising at least one hydroxyl group,

**characterized in that** the molar ratio of the acyl residues $R^4$ to $R^5$ is in a range of 95:5 to 5:95.

**2.** Polyglycerol partial ester according to Claim 1, **characterized in that** $R^4$ and $R^5$ are acyl residues of fatty acids.

**3.** Polyglycerol partial ester according to Claim 1 or 2, **characterized in that** at least 50 mol% of the $R^4$ acyl residues are selected from capryloyl, caproyl and lauroyl residues, based on all $R^4$ residues in the polyglycerol partial ester.

**4.** Polyglycerol partial ester according to at least one of the preceding claims, **characterized in that** $R^5$ is selected from ricinoyl and hydroxystearoyl residues, their oligomers and mixtures thereof.

**5.** Polyglycerol partial ester according to at least one of the preceding claims, **characterized in that** at least 90 mol% of the $R^5$ acyl residues comprise ricinoyl residues or a mixture of ricinoyl and hydroxystearoyl residues, based on all $R^5$ residues in the polyglycerol partial ester.

**6.** Polyglycerol partial ester according to at least one of Claims 1 to 4, **characterized in that** $R^5$ is selected from ricinoyl residues.

**7.** Polyglycerol partial ester according to at least one of the preceding claims, **characterized in that** the weight ratio of the polyglyceryl residue to the sum total of the acyl residues $R^4$ and $R^5$ is 85:15 to 55:45.

**8.** Method for preparing polyglycerol partial esters according to Claim 1 comprising the method steps of:

A) providing a polyglycerol having a mean degree of polymerisation n = 2 to 16,
B) acylation of some of the hydroxyl groups of the polyglycerol with
at least one first carboxylic acid derivative of one or more first, saturated or unsaturated carboxylic acids having 6 - 22 carbon atoms comprising no hydroxyl groups and
at least one second carboxylic acid derivative of one or more second, saturated or unsaturated carboxylic acids having 6 - 22 carbon atoms comprising at least one hydroxyl group or an oligomer of the second carboxylic acid, in which the carboxylic acid derivatives are selected from carboxylic acids and carboxylic esters,

wherein the molar ratio of the acyl residues of the first carboxylic acid derivative used in method step B) to those of the second carboxylic acid derivative is in a range of 95:5 to 5:95.

**9.** Method according to Claim 8 or 9, **characterized in that** in method step B)

at least one first carboxylic acid and at least one second carboxylic acid,
at least one first carboxylic ester and at least one second carboxylic acid,
at least one first carboxylic acid and at least one second carboxylic ester,
at least one first carboxylic ester and at least one second carboxylic ester,
at least one first carboxylic acid and at least one first carboxylic ester and at least one second carboxylic acid,
at least one first carboxylic acid and at least one first carboxylic ester and at least one second carboxylic ester,
at least one first carboxylic acid and at least one second carboxylic acid and at least one second carboxylic ester,
at least one first carboxylic ester and at least one second carboxylic acid and at least one second carboxylic ester,
or
at least one first carboxylic acid and at least one first carboxylic ester and at least one second carboxylic acid and at least one second carboxylic ester,
are used.

**10.** Method according to Claim 8 or 9, **characterized in that** at least 50 mol% of the first carboxylic acids are selected from caprylic acid, capric acid and lauric acid, based on the acyl residues of all the first carboxylic acid derivatives.

**11.** Method according to at least one of Claims 8 to 10, **characterized in that** at least 90 mol% of the second carboxylic acids are selected from ricinoleic acid and hydroxystearic acid, based on the acyl residues of all the second carboxylic acid derivatives.

**12.** Method according to at least one of Claims 8 to 11, **characterized in that** at least 90 mol% of the second carboxylic acids comprise ricinoleic acid and hydroxystearic acid, and the second carboxylic acids have a molar ratio of ricinoleic acid residues to hydroxystearic acid residues in a range of 100 to 0.1 to 50 to 50, based on the acyl residues of all the second carboxylic acid derivatives.

**13.** Method according to at least one of Claims 8 to 12, **characterized in that** the weight ratio of the polyglycerol to the sum total of the acyl residues of the first and second carboxylic acid derivatives used is 85:15 to 55:45.

**14.** Method according to at least one of Claims 8 to 13, **characterized in that** the molar ratio of the acyl residues of saturated to unsaturated carboxylic acid derivatives used in method step B) is 99:1 - 1:99.

**15.** Formulation comprising at least one polyglycerol partial ester according to at least one of Claims 1 to 7.

**16.** Formulation according to Claim 15, comprising

0.1% by weight to 40% by weight of polyglycerol partial ester according to at least one of Claims 1 to 7
0.01% by weight to 40% by weight of oil-soluble substance and
10% by weight to 98% by weight of water.

**17.** Use of at least one polyglycerol partial ester according to at least one of Claims 1 to 7 for solubilizing at least one oil-soluble substance in water.

## Revendications

**1.** Ester partiel de polyglycérine de formule générale I

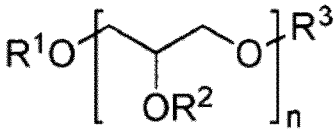

Formule générale I

avec

n = 2 à 16, de préférence 4 à 14, de manière particulièrement préférée 5 à 11,
$R^1$, $R^2$, $R^3$ = indépendamment les uns des autres, de manière identique ou différente, choisis parmi H, $R^4$ et $R^5$,

$R^4$ = radical acyle saturé ou insaturé ne contenant pas de groupe hydroxyle, de 6 à 22 atomes C, de préférence de 8 à 18 atomes C,
$R^5$ = radical acyle saturé ou insaturé contenant au moins un groupe hydroxyle, de 6 à 22 atomes C, de préférence de 14 à 22 atomes C, ou radical acyle d'un oligomère de radicaux acyle saturés ou insaturés contenant au moins un groupe hydroxyle, de 6 à 22 atomes C, de préférence de 14 à 22 atomes C,
**caractérisé en ce que** le rapport molaire entre les radicaux acyle $R^4$ et $R^5$ se situe dans une plage allant de 95:5 à 50:50.

2. Ester partiel de polyglycérine selon la revendication 1, **caractérisé en ce que** $R^4$ et $R^5$ sont des radicaux acyle d'acides gras.

3. Ester partiel de polyglycérine selon la revendication 1 ou 2, **caractérisé en ce que**, par rapport à tous les radicaux $R^4$ dans l'ester partiel de polyglycérine, au moins 50 % en moles des radicaux acyle $R^4$ sont choisis parmi les radicaux capryloyle, caproyle et lauroyle.

4. Ester partiel de polyglycérine selon au moins l'une quelconque des revendications précédentes, **caractérisé en ce que** $R^5$ est choisi parmi les radicaux ricinoyle et hydroxystéaroyle, leurs oligomères et leurs mélanges.

5. Ester partiel de polyglycérine selon au moins l'une quelconque des revendications précédentes, **caractérisé en ce que**, par rapport à tous les radicaux $R^5$ dans l'ester partiel de polyglycérine, au moins 90 % en moles des radicaux acyle $R^5$ sont constitués par des radicaux ricinoyle ou un mélange de radicaux ricinoyle et hydroxystéaroyle.

6. Ester partiel de polyglycérine selon au moins l'une quelconque des revendications 1 à 4, **caractérisé en ce que** $R^5$ est choisi parmi les radicaux ricinoyle.

7. Ester partiel de polyglycérine selon au moins l'une quelconque des revendications précédentes, **caractérisé en ce que** le rapport en poids entre le radical polyglycéryle et la somme des radicaux acyle $R^4$ et $R^5$ est de 85:15 à 55:45.

8. Procédé de fabrication d'esters partiels de polyglycérine selon la revendication 1, comprenant les étapes de procédé suivantes :

   A) la préparation d'une polyglycérine ayant un degré de polymérisation moyen n = 2 à 16,
   B) l'acylation d'une partie des groupes hydroxy de la polyglycérine avec au moins un premier dérivé d'acide carboxylique d'un ou de plusieurs premiers acides carboxyliques saturés ou insaturés ne contenant pas de groupe hydroxy, de 6 à 22 atomes C, et
   au moins un deuxième dérivé d'acide carboxylique d'un ou de plusieurs deuxièmes acides carboxyliques saturés ou insaturés contenant au moins un groupe hydroxy, de 6 à 22 atomes C, ou d'un oligomère du deuxième acide carboxylique,
   les dérivés d'acides carboxyliques étant choisis parmi les acides carboxyliques et les esters d'acides carboxyliques,

   le rapport molaire entre les radicaux acyle du premier dérivé d'acide carboxylique utilisé à l'étape de procédé B) et ceux du deuxième dérivé d'acide carboxylique se situant dans une plage allant de 95:5 à 50:50.

9. Procédé selon la revendication 8 ou 9, **caractérisé en ce qu'**à l'étape de procédé B),
   au moins un premier acide carboxylique et au moins un deuxième acide carboxylique,
   au moins un premier ester d'acide carboxylique et au moins un deuxième acide carboxylique,
   au moins un premier acide carboxylique et au moins un deuxième ester d'acide carboxylique,
   au moins un premier ester d'acide carboxylique et au moins un deuxième ester d'acide carboxylique,
   au moins un premier acide carboxylique et au moins un premier ester d'acide carboxylique et au moins un deuxième acide carboxylique,
   au moins un premier acide carboxylique et au moins un premier ester d'acide carboxylique et au moins un deuxième ester d'acide carboxylique,
   au moins un premier acide carboxylique et au moins un deuxième acide carboxylique et au moins un deuxième ester d'acide carboxylique,
   au moins un premier ester d'acide carboxylique et au moins un deuxième acide carboxylique et au moins un deuxième ester d'acide carboxylique,
   ou

au moins un premier acide carboxylique et au moins un premier ester d'acide carboxylique et au moins un deuxième acide carboxylique et au moins un deuxième ester d'acide carboxylique,
sont utilisés.

**10.** Procédé selon la revendication 8 ou 9, **caractérisé en ce que**, par rapport aux radicaux acyle de tous les premiers dérivés d'acides carboxyliques, au moins 50 % en moles des premiers acides carboxyliques sont choisis parmi l'acide caprylique, l'acide caprique et l'acide laurique.

**11.** Procédé selon au moins l'une quelconque des revendications 8 à 10, **caractérisé en ce que**, par rapport aux radicaux acyle de tous les deuxièmes dérivés d'acides carboxyliques, au moins 90 % en moles des deuxièmes acides carboxyliques sont choisis parmi l'acide ricinoléique et l'acide hydroxystéarique.

**12.** Procédé selon au moins l'une quelconque des revendications 8 à 11, **caractérisé en ce que**, par rapport aux radicaux acyle de tous les deuxièmes dérivés d'acides carboxyliques, au moins 90 % en moles des deuxièmes acides carboxyliques sont constitués par de l'acide ricinoléique et de l'acide hydroxystéarique, et les deuxièmes acides carboxyliques présentent un rapport molaire entre les radicaux acide ricinoléique et les radicaux acide hydroxystéarique dans une plage allant de 100 sur 0,1 à 50 sur 50.

**13.** Procédé selon au moins l'une quelconque des revendications 8 à 12, **caractérisé en ce que** le rapport en poids entre la polyglycérine et la somme des radicaux acyle des premiers et deuxièmes dérivés d'acides carboxyliques utilisés est de 85:15 à 55:45.

**14.** Procédé selon au moins l'une quelconque des revendications 8 à 13, **caractérisé en ce que** le rapport molaire entre les radicaux acyle des dérivés d'acides carboxyliques saturés et insaturés qui sont utilisés à l'étape de procédé B) est de 99:1 à 1:99.

**15.** Formulation contenant au moins un ester partiel de polyglycérine selon au moins l'une quelconque des revendications 1 à 7.

**16.** Formulation selon la revendication 15, contenant : 0,1 % en poids à 40 % en poids d'ester partiel de polyglycérine selon au moins l'une quelconque des revendications 1 à 7,
0,01 % en poids à 40 % en poids d'une substance soluble dans les huiles, et
10 % en poids à 98 % en poids d'eau.

**17.** Utilisation d'au moins un ester partiel de polyglycérine selon au moins l'une quelconque des revendications 1 à 7 pour la solubilisation d'au moins une substance soluble dans les huiles dans l'eau.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- JP 2008119568 A
- WO 2007027447 A
- EP 2273966 A1


### In der Beschreibung aufgeführte Nicht-Patentliteratur


- **CASSEL et al.** Original synthesis of linear, branched and cyclic oligoglycerol Standards. *J. Org. Chem.,* 2001, 875-896
- **HANDBÜCHER ; ZUM BEISPIEL K. ; SCHRADER.** Grundlagen und Rezepturen der Kosmetika. Hüthig Buch Verlag Heidelberg, 329-341